# EUROPEAN PATENT SPECIFICATION

(11) **EP 1 524 286 B1**
(45) Date of publication and mention of the grant of the patent: **16.12.2009**
(21) Application number: 04022032.9
(22) Date of filing: 16.09.2004
(51) Int. Cl.: C08G 61/12, C09K 19/38, C07D 495/04

(54) **Poly(benzodithiophenes)**
Polybenzodithiophene
polybenzodithiophènes

(30) Priority: 15.10.2003 EP 03023339
(43) Date of publication of application: 20.04.2005
(73) Proprietor: Merck Patent GmbH, 64293 Darmstadt (DE)
(72) Inventor: Heeney, Martin, Southampton SO14 6TQ (GB); Bailey, Clare, Midanbury Southampton Hampshire SO18 4PP (GB); Tierney, Steven, Southampton SO15 7QW (GB); Zhang, Weimin, Southampton SO16 7DE (GB); McCulloch, Iain, Dr., Southampton SO53 4LG (GB)

(56) References cited:
- EP-A- 1 300 430
- EP-A- 1 357 163
- PATENT ABSTRACTS OF JAPAN vol. 2000, no. 02, 29 February 2000 (2000-02-29) & JP 11 322906 A (YAMAMOTO RYUICHI), 26 November 1999 (1999-11-26)
- SHIRAISHI ET AL.: "New pi-conjugated polymers constituted of dialkoxybenzodithiophene units: synthesis and electronic properties" SYNTHETIC MATERIALS, vol. 130, 2002, pages 139-147, XP002314286

## Description

### Field of Invention

The invention relates to novel poyl(benzodithiophenes). The invention further relates to their use as semiconductors or charge transport materials, in optical, electro-optical or electronic devices like for example liquid crystal displays, optical films, organic field effect transistors (FET or OFET) for thin film transistor liquid crystal displays and integrated circuit devices such as RFID tags, electroluminescent devices in flat panel displays, and in photovoltaic and sensor devices. The invention further relates to a field effect transistor, light emitting device or ID tag comprising the novel polymers.

### Background and Prior Art

Organic materials have recently shown promise as the active layer in organic based thin film transistors and organic field effect transistors [see H. E. Katz, Z. Bao and S. L. Gilat, *Acc. Chem. Res.*, 2001, **34**, 5, 359]. Such devices have potential applications in smart cards, security tags and the switching element in flat panel displays. Organic materials are envisaged to have substantial cost advantages over their silicon analogues if they can be deposited from solution, as this enables a fast, large-area fabrication route.

The performance of the device is principally based upon the charge carrier mobility of the semi-conducting material and the current on/off ratio, so the ideal semiconductor should have a low conductivity in the off state, combined with a high charge carrier mobility (> 1 x 10⁻³ cm² V⁻¹ s⁻¹). In addition, it is important that the semi-conducting material is relatively stable to oxidation i.e. it has a high ionisation potential, as oxidation leads to reduced device performance.

One of the best semi-conducting polymers currently available is regioregular poly(alkyl)thiophene (PAT), with a mobility around 0.1 cm²V⁻¹s⁻¹. For example, regular poly(3-hexylthiophene) has been reported with a high charge carrier mobility between 1 x 10⁻⁵ and 4.5 x 10⁻² cm² V⁻¹ s⁻¹, but with a rather low current on/off ratio between 10 and 10³ [see Z. Bao et al., Appl. Phys. Lett. 1996, 69, 4108]. In general, poly(3-alkylthiophenes) show improved solubility and are able to be solution processed to fabricate large area films. However, poly(3-alkylthiophenes) have relatively low ionisation potentials and are susceptible to doping in air [see H. Sirringhaus et al., Adv. Solid State Phys. 1999, 39, 101].

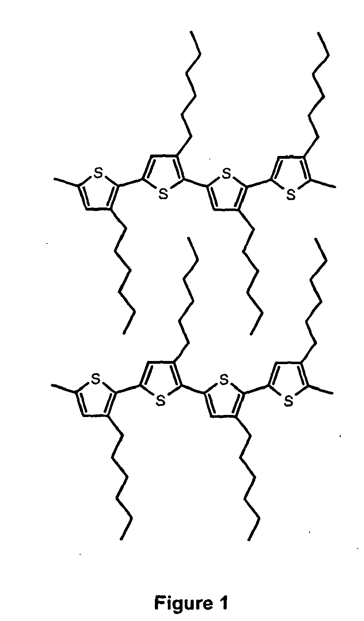

There are a number of features in PAT that contribute to its high charge carrier mobility. Firstly, the polymer chains of regioregular PAT are able to pack via interdigitation of their side-chains, as schematically depicted in **Figure 1**. The side-chains are necessary to provide polymers that are soluble. This results in the formation of lamellar sheets of polymers, such a arrangement is beneficial to the charge-hopping mechanism with which charge is carried in organic materials [see P. J. B. H. Sirringhaus, R. H. Friend, M. M. Nielsen, K. Bechgaard, B. M. W. Langeveld-Voss, A. J. H. Spiering, R. A. J. Janssen, E. W. Meijer, P. Herwig & D. M. de Leeuw, NATURE, 1999, 401, 685]. Secondly, PAT contains an abundance of sulfur atoms in the thiophene rings. The presence of sulfur atoms has been shown to be beneficial to charge transport, the exact mechanism is not known, but it is speculated that interaction of the sulfur d-orbitals on adjacent polymer chains facilitates the charge hopping mechanism. The major drawback of PAT, however, is that the material is oxidatively unstable. This means that the material chemically degrades in the presence of oxygen, leading to low shelf stability, and secondly the material is susceptable to doping by oxygen which results in high transistor off currents and poor transistor performance. The oxidative instability of PAT is due to the presence of many electron-rich thiophene rings in the polymer backbone, which results in a high HOMO level (around -4.9 eV).

Incorporation of benzodithiophene units in a PAT-backbone results in a polymer backbone that is less electron rich than the equivalent all-thiophene polymer, since benzene is much less electron rich than thiophene.

Benzo[1,2-b:4,5-b'] dithiophene, hereinafter also shortly referred to as benzodithiophene or BDT, with the following structure (1) 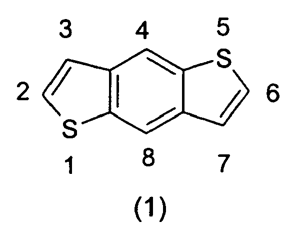 has been reported in the literature to have a high charge carrier mobility and to be useful as organic semiconductor. BDT monomers or dimers, oligo- or polymers formed thereof and their use as an organic semiconductors have been described for example in Kossmehl et al., Makromol. Chem. 1983, 184(3), 627-50, Katz et al., J. Mater. Chem. 1997, 7(3), 369-76, Laquindanum et al., Adv. Mater. 1997, 9(1), 36-39 and in US 5,625,199. For example, bis(benzodithiophene) (2) is stable up to 400°C in air [see H. E. Katz; Z. Bao; S. L. Gilat, *Accounts of Chemical Research,* 2001, **34**, 359].

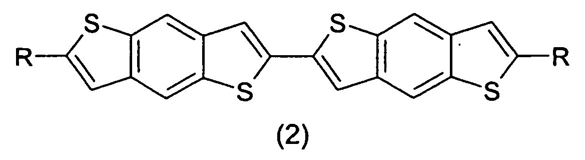

In particular the dimeric bisbenzodithiophene (2) has been shown to exhibit high charge carrier mobilities of 0.04 cm²V⁻¹s⁻¹. Its structure has flatter conformation than e.g. α-sexithiophene with comparable size. This enables compressed molecular packing and strong intermolecular interactions, which is favourable for compact stacking of the material and results in π-π-overlap and hence makes this compound an effective charge transport materials with high carrier mobilities. However, bisbenzodithiophene has a very high melting point over 400 °C and very low solubility in organic solvents, so that it cannot be readily solution processed and can only be vacuum deposited.

To date some example's of a poly(benzodithiophene) substituted in the 4,8 positions have been reported. Shiraishi and Yamamoto reported polymers and co-polymers based upon alkoxy substituted BDT (3) [see K. Shiraishi; T. Yamamoto, Synthetic Metals, 2002, 130, 139-147].

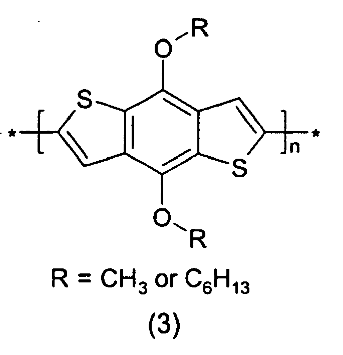

However, these polymers were poorly soluble and were only investigated as potential conductive materials after doping. Only alkoxy substituted polymers were described, however, these are undesirable for semiconducting materials since the electron donating alkoxy groups results in an increase of the HOMO level of the polymer, and subsequent stability problems. Additionally the routes described to these polymers are not amenable to the synthesis of the alkyl substituted polymers described here. Pomerantz et al reported a polymer whereupon benzodithiophene was polymerised through the 4,8 positions [see M. Pomerantz; J. Wang; S. Seong; K. P. Starkey; L. Nguyen; D. S. Marynick, Macromolecules, 1994, 27, 7478-7485]. However, no examples of alkyl substituted polymers or co-polymers have been described.

It was an aim of the present invention to provide new organic materials for use as semiconductors or charge transport materials, which are easy to synthesise, have high charge mobility and good processability. The materials should be easily processable to form thin and large-area films for use in semiconductor devices. In particular the materials should be oxidatively stable, but retain or even improve the desirable properties of PAT. Another aim of the invention was to provide BDT materials that are more easily processible in the manufacture of semiconductor devices, are stable and allow easy synthesis also at large scale.

It was found that the above aims can be achieved by providing poly(benzodithiophenes) according to the present invention. These polymers still possess alkyl chains perpendicular to the polymer backbone that are able to both solubilise the polymer in organic solvents and additionally are able to form closely packed interdigitated structures. Two positions are available for the alkyl chains in the BDT polymers according to this invention: substitution in 4,8-position (3) or substitution in 3,7-position (4).

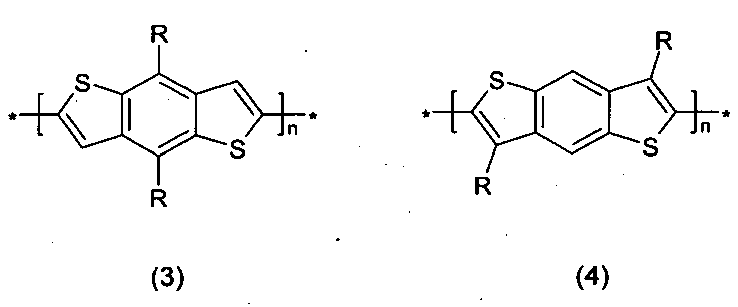

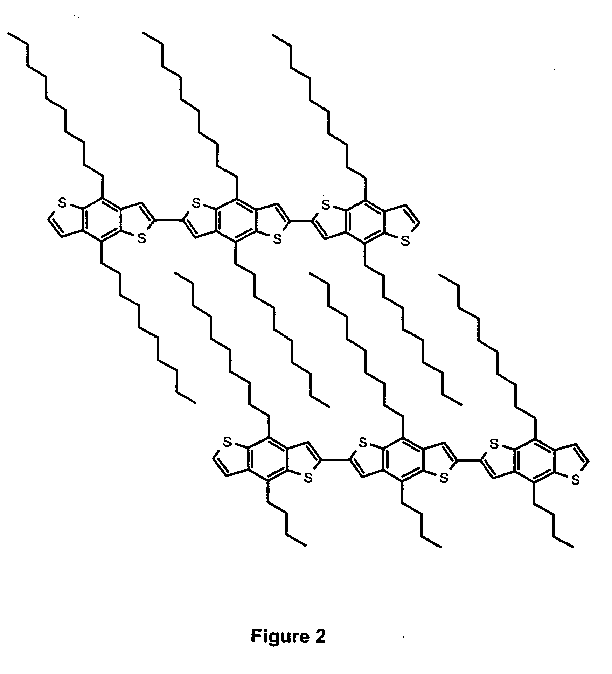

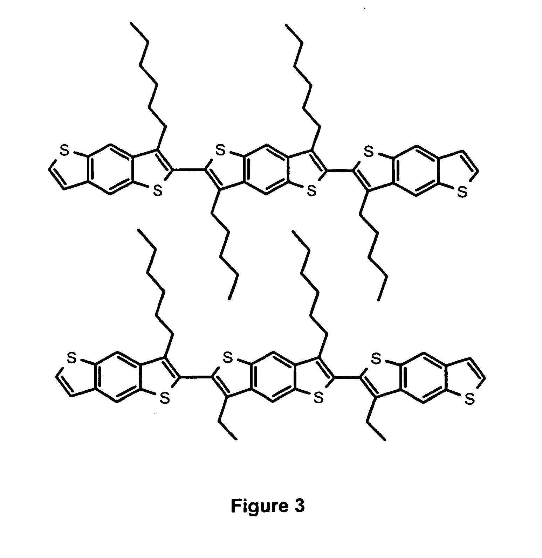

Both these positions can afford closely packed structures, as schematically depicted in **Figure 2** for the homo-polymer substituted in the 4,8 positions and in **Figure 3** for the polymer substituted in the 3,7- positions. Furthermore, like PAT, the benzodithiophene polymers have sulfur atoms in the polymer backbone. An additional advantage of the poly(benzodithiophenes) according to the present invention is that the monomers from which the polymers are synthesised are symmetrical, therefore there are no problems regarding the regioregularity of the resulting polymer. This simplifies the synthesis.

JP 11-322906 A discloses benzodithiophene-4,8-diones, but does not disclose benzodithiophene polymers that are substituted in 4- and 8-position by groups R¹ and R² as defined in the present application.

Pomerantz et al., Macromolecules 1994, 27, 7478-7485 discloses polymers with benzodithiophene-4,8-diyl units, but does not disclose polymers with benzodithiophene-2,6-diyl units as defined in the present application.

### Summary of the Invention

The invention relates to mono-, oligo- or polymers comprising at least one benzodithiophene (BDT) group that is substituted in 3- and 7-position and/or in 4- and 8-position, which are selected from formulae I, I1 and I1a shown below.

The invention further relates to a semiconductor or charge transport material, component or device comprising at least one mono-, oligo- or polymer as defined above.

The invention further relates to the use of polymers according to the invention as semiconductors or charge transport materials, in particular in optical, electrooptical or electronic devices, like for example in field effect transistors (FET) as components of integrated circuitry, as thin film transistors in flat panel display applications or for Radio Frequency Identification (RFID) tags, or in semiconducting components for displays or organic light emitting diodes (OLED), including both the charge transport and electroluminescent layers.

The invention further relates to the use of the novel mono-, oligo- and polymers according to the present invention as electroluminescent materials, for OLED applications such as electroluminescent displays or backlights of displays, in photovoltaic or sensor devices, as electrode materials in batteries, as photoconductors, for electrophotographic applications like electrophotographic recording, for organic memory devices, for detecting and discriminating DNA sequences, and as alignment layer in LCD or OLED devices.

The invention further relates to an optical, electrooptical or electronic device, FET, integrated circuit (IC), TFT, OLED or alignment layer comprising a semiconducting or charge transport material, component or device according to the invention.

The invention further relates to a TFT or TFT array for flat panel displays, radio frequency identification (RFID) tag, electroluminescent display or backlight comprising a semiconducting or charge transport material, component or device or a FET, IC, TFT or OLED according to the invention.

The invention further relates to a security marking or device comprising a FET or an RFID tag according to the invention.

### Detailed Description of the Invention

The mono-, oligo and polymers according to the invention are especially useful as charge transport semiconductors in that they have high carrier mobilities. Particularly preferred are polymers wherein the BDT group is substituted by one or more alkyl or fluoroalkyl groups. The introduction of fluoroalkyl and alkyl side chains into the BDT group improves their solubility and therefore their solution processibility. Furthermore, the presence of fluoroalkyl side chains also renders these materials effective as *n*-type semiconductors. The electron-withdrawing nature of the fluoroalkyl substituents will also lower the HOMO further and result in a more stable material, which is less susceptible to oxidation.

Copolymerisation of BDT with functionalised aromatic or unsaturated comonomers can further improve the solubility and the charge transport properties. Variation of the aromatic comonomers provides a method of tailoring the band gap of the polymers. This will lead to better stability and higher charge carrier mobility.

The invention relates to oligo- and polymers comprising one or more identical or different recurring units of formula I 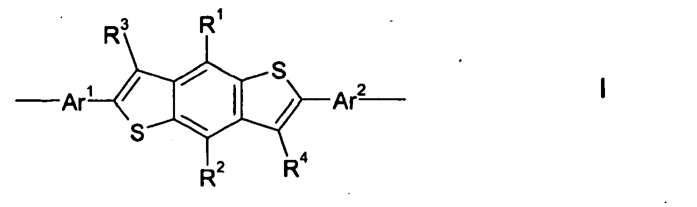 wherein
- R¹ to R⁴: are independently of each other H, halogen or straight chain, branched or cyclic alkyl with 1 to 20 C-atoms, which may be unsubstituted, mono- or poly-substituted by F, Cl, Br, I or CN, it being also possible for one or more non-adjacent CH₂ groups to be replaced, in each case independently from one another, by -O-, -S-, -NH-, -NR⁰-, -SiR⁰R⁰⁰-, -CO-, -COO-, -OCO-, -OCO-O-, -S-CO-, -CO-S-, -CH=CH- or -C≡C- in such a manner that O and/or S atoms are not linked directly to one another, or optionally substituted aryl or heteroaryl, or P-Sp-,
- P: is a polymerisable or reactive group,
- Sp: is a spacer group or a single bond,
- R⁰ and R⁰⁰: are independently of each other H or alkyl with 1 to 12 C-atoms, and
- Ar¹ and Ar²: are independently of each other -CX¹=CX²-, -C≡C-, an arylene or heteroarylene group that is optionally substituted with one or more groups R¹, or a single bond,
- X¹ and X²: are independently of each other H, F, Cl or CN,
with the provisos that
a) in at least one recurring unit R¹ and R² are different from H and/or R³ and R⁴ are different from H,
b) R¹ and R² do not at the same time denote an alkoxy group, and wherein the number of recurring units is from 10 to 5000.

Particularly preferred are oligo and polymers of formula l1 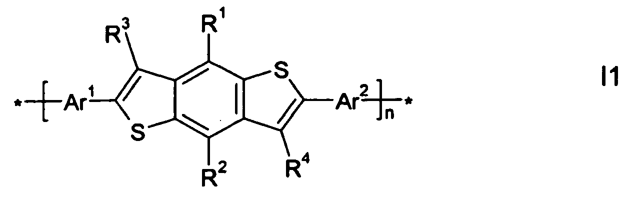 wherein R¹, R², R³, R⁴, Ar¹ and Ar² have independently of each other one of the meanings of formula I, n is an integer from 10 to 5000, and wherein the recurring units may be identical or different.

The oligo- and polymers of formula I1 are preferably selected of formula I1a 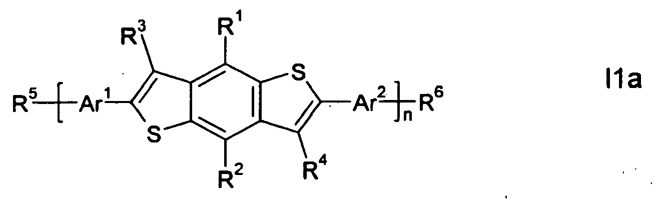 wherein R¹, R², R³, R⁴, Ar¹, Ar² and n have independently of each other one of the meanings of formula I,
- R⁵ and R⁶: are independently of each other H, halogen, -Sn(R⁰)₃, -B(OR')(OR"), -CH₂Cl, -CHO, -CH=CH₂, -SiR⁰R⁰⁰R⁰⁰⁰⁰, optionally substituted aryl or heteroaryl or P-Sp-,
- R⁰⁰⁰: has one of the meanings of R⁰ in formula I,
- R' and R": are independently of each other H or alkyl with 1 to 12 C-atoms, or OR' and OR" together with the boron atom form a cyclic group having 2 to 20 C atoms, and
- P, Sp, R⁰ and R⁰⁰: have the meanings of formula I.

The invention further relates to monomers of formula 11 a 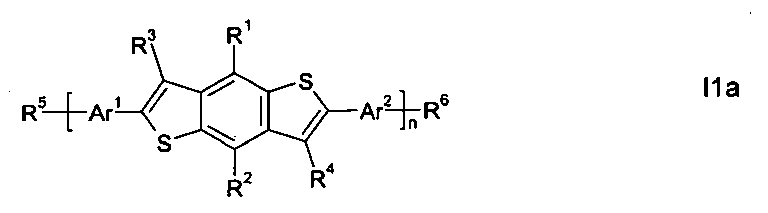 wherein R¹, R², R³, R⁴, Ar¹ and Ar² have independently of each other one of the meanings of formula 11, n is 1, and R⁵ and R⁶ are selected from Cl, Br, I, -Sn(R⁰)₃, -B(OR')(OR"), -CH₂Cl, - CHO and -SiR⁰R⁰⁰R⁰⁰⁰.

Especially preferred are mono-, oligo-, and polymers of formula I, I1 and I1a having identical recurring units.

Further preferred are mono-, oligo-, and polymers of formula I, I1 and I1a wherein R¹ and R², or R³ and R⁴, respectively, are identical groups.

Especially preferred are oligo-, and polymers of formula I, I1 and I1a having a degree of polymerisation (number n of recurring units) from 100 to 1000.

Further preferred are mono-, oligo-, and polymers of formula I, I1 and I1a having a molecular weight (Mw) from 5000 to 300,000, in particular from 20,000 to 100,000.

Especially preferred are regioregular polymers of formula I, I1 and I1a, in particular with a high regioregularity of head-to-tail (HT) couplings. For example, in case of 3,7-substituted polyBDTs, the polymers preferably exhibit a high degree of HT-couplings of the following type

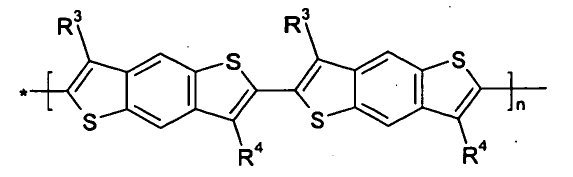

The regioregularity in these polymers is preferably at least 90 %, in particular 95% or more, very preferably 98% or more, most preferably from 99 to 100%.

Regioregular polymers are advantageous as they show strong interchain pi-pi-stacking interactions and a high degree of crystallinity, making them effective charge transport materials with high carrier mobilities.

Further preferred are mono-, oligo- and polymers of formula I, I1 and I1a comprising at least one reactive group P that is capable of a polymerisation or crosslinking reaction.

Further preferred are mono-, oligo- and polymers of formula I, I1 and I1a that are mesogenic or liquid crystalline, in particular polymers forming calamitic phases, and reactive mesogens of formula I, I1 and I1a comprising one or more groups P-Sp-, forming calamitic phases.

Further preferred are mono-, oligo- and polymers of formula I, I1 and I1a shown above and below wherein
- Ar¹ and/or Ar² is thiophene-2,5-diyl that is optionally substituted,
- one of Ar¹ and Ar² is a single bond,
- both Ar¹ and Ar² are a single bond,
- Ar¹ and/or Ar² is CX¹ =CX²,
- R¹ and R² are selected from C₁-C₂₀-alkyl that is optionally substituted with one or more fluorine atoms, C₁-C₂₀-alkenyl, C₁-C₂₀-alkynyl, C₁-C₂₀-thioalkyl, C₁-C₂₀-silyl, C₁-C₂₀-ester, C₁-C₂₀-amino, C₁-C₂₀-fluoroalkyl, and optionally substituted aryl or heteroaryl, very preferably C₁-C₂₀-alkyl or C₁-C₂₀-fluoroalkyl, very preferably with R³ and R⁴ being H,
- R³ and R⁴ are selected from C₁-C₂₀-alkyl that is optionally substituted with one or more fluorine atoms, C₁-C₂₀-alkenyl, C₁-C₂₀-alkynyl, C₁-C₂₀-thioalkyl, C₁-C₂₀-silyl, C₁-C₂₀-ester, C₁-C₂₀-amino, C₁-C₂₀-fluoroalkyl, and optionally substituted aryl or heteroaryl, very preferably C₁-C₂₀-alkyl or C₁-C₂₀-fluoroalkyl, very preferably with R¹ and R² being H,
- R¹ and R² are different from an alkoxy group,
- R³ and R⁴ are different from an alkoxy group,
- R⁵ and R⁶ are selected from Cl, Br, I, -Sn(R⁰)₃, -B(OR')(OR"), -CH₂Cl, -CHO, -SiR⁰R⁰⁰R⁰⁰⁰⁰ and n is 1.

If Ar¹ or Ar² is arylene or heteroarylene, it is preferably a mono-, bi- or tricyclic aromatic or heteroaromatic group with up to 25 C atoms, wherein the rings can be fused, and in which the heteroaromatic group contains at least one hetero ring atom, preferably selected from N, O and S. It is optionally substituted with one or more of F, Cl, Br, I, CN, and straight chain, branched or cyclic alkyl having 1 to 20 C atoms, which is unsubstituted, mono- or poly-substituted by F, Cl, Br, I, -CN or -OH, and in which one or more non-adjacent CH₂ groups are optionally replaced, in each case independently from one another, by -O-, -S-, -NH-, -NR⁰-, -SiR⁰R⁰⁰-, -CO-, -COO-, OCO-, -OCO-O, -S-CO-, -CO-S-,-CH=CH- or -C≡- in such a manner that O and/or S atoms are not linked directly to one another.

Preferred groups arylene or heteroarylene groups are selected from phenyl in which, in addition, one or more CH groups may be replaced by N, or naphthalene, alkyl fluorene or oxazole, wherein all these groups are optionally mono- or polysubstituted with L, wherein L is F, Cl, Br, or an alkyl, alkoxy, alkylcarbonyl, alkylcarbonyloxy or alkoxycarbonyl group with 1 to 12 C atoms, wherein one or more H atoms are optionally replaced by F or Cl. Further preferred groups are thiophene, thienothiophene and dithienothiophene which are substituted by one or more halogen, in particular fluorine, atoms.

Especially preferred arylene or heteroarylene groups groups are 1,4-phenylene, fluorinated 1,4-phenylene, 2,5-pyridine, 2,5-pyrimidine, p,p'-biphenyl, naphthalene-2,6-diyl, thiophene-2,5-diyl, fluorinated or alkylated thiophene-2,5-diyl, fluorinated benzo[1,2-b:4,5-b']dithiophene, 2,5-thiazole, 2,5-thiadiazole, 2,5-oxazole and 2,5-oxadiazole, all of which are unsubstituted, mono- or polysubstituted with L as defined above.

If one of R¹⁻⁶ is aryl or heteroaryl, it is preferably a mono-, bi- or tricyclic aromatic or heteroaromatic group with up to 25 C atoms, wherein the rings can be fused, and in which the heteroaromatic group contains at least one hetero ring atom, preferably selected from N, O and S. It is optionally substituted with one or more of F, Cl, Br, I, CN, and straight chain, branched or cyclic alkyl having 1 to 20 C atoms, which is unsubstituted, mono- or poly-substituted by F, Cl, Br, I, -CN or -OH, and in which one or more non-adjacent CH₂ groups are optionally replaced, in each case independently from one another, by-O-, -S-, -NH-, -NR⁰-, -SiR⁰R⁰⁰-, -CO-, -COO-, OCO-, -OCO-O, -S-CO-, -CO-S-,-CH=CH- or -C ≡C- in such a manner that O and/or S atoms are not linked directly to one another.

Especially preferred aryl and heteroaryl groups are phenyl, fluorinated phenyl, pyridine, pyrimidine, biphenyl, naphthalene, fluorinated thiophene, benzo[1,2-b:4,5-b']dithiophene, thiazole and oxazole, all of which are unsubstituted, mono- or polysubstituted with L as defined above.

If one of R¹⁻⁴ is an alkyl or alkoxy radical, i.e. where the terminal CH₂ group is replaced by -O-, this may be straight-chain or branched. It is preferably straight-chain, has 2 to 8 carbon atoms and accordingly is preferably ethyl, propyl, butyl, pentyl, hexyl, heptyl, octyl, ethoxy, propoxy, butoxy, pentoxy, hexyloxy, heptoxy, or octoxy, furthermore methyl, nonyl, decyl, undecyl, dodecyl, tridecyl, tetradecyl, pentadecyl, nonoxy, decoxy, undecoxy, dodecoxy, tridecoxy or tetradecoxy, for example.

Fluoroalkyl or fluorinated alkyl or alkoxy is preferably straight chain (O)CᵢF₂ᵢ₊₁, wherein i is an integer from 1 to 20, in particular from 1 to 15, very preferably (O)CF₃, (O)C₂F₅, (O)C₃F₇, (O)C₄F₉, (O)C₅F₁₁, (O)C₆F₁₃, (O)C₇F₁₅ or (O)C₈F₁₇, most preferably (O)C₆F₁₃.

CX¹=CX² is preferably -CH=CH-, -CH=CF-, -CF=CH-, -CF=CF-, -CH=C(CN)- or -C(CN)=CH-.

Halogen is preferably F, Br, Cl or I.

Hetero atoms are preferably selected from N, O and S.

The polymerisable or reactive group P is preferably selected from CH₂=CW¹-COO-, 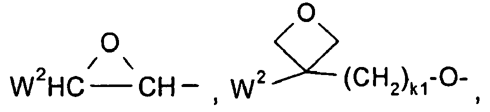 CH₂=CW²- (O)ₖ₁-, CH₃-CH=CH-O-, (CH₂=CH)₂CH-OCO-, (CH₂=CH)₂CH-O-, (CH₂=CH-CH₂)₂CH-OCO-, (CH₂=CH-CH₂)₂N-, HO-CW²W³-, HS-CW²W³-, HW²N-, HO-CW²W³-NH-, CH₂=CW¹-CO-NH-, CH₂=CH-(COO)ₖ₁-Phe-(O)ₖ₂-, Phe-CH=CH-, HOOC-, OCN-, and W⁴W⁵W⁶Si-, with W¹ being H, Cl, CN, phenyl or alkyl with 1 to 5 C-atoms, in particular H, Cl or CH₃, W² and W³ being independently of each other H or alkyl with 1 to 5 C-atoms, in particular methyl, ethyl or n-propyl, W⁴, W⁵ and W⁶ being independently of each other Cl, oxaalkyl or oxacarbonylalkyl with 1 to 5 C-atoms, Phe being 1,4-phenylene and k₁ and k₂ being independently of each other 0 or 1.

Especially preferred groups P are CH₂=CH-COO-, CH₂=C(CH₃)-COO-, CH₂=CH-, CH₂=CH-O-, (CH₂=CH)₂CH-OCO-, (CH₂=CH)₂CH-O-, and

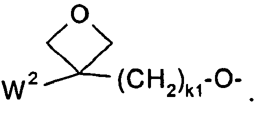

Very preferred are acrylate and oxetane groups. Oxetanes produce less shrinkage upon polymerisation (cross-linking), which results in less stress development within films, leading to higher retention of ordering and fewer defects. Oxetane cross-linking also requires cationic initiator, which unlike free radical initiator is inert to oxygen.

As for the spacer group Sp all groups can be used that are known for this purpose to the skilled in the art. The spacer group Sp is preferably of formula Sp'-X, such that P-Sp- is P-Sp'-X-, wherein
- Sp': is alkylene with up to 20 C atoms which may be unsubstituted, mono- or poly-substituted by F, Cl, Br, I or CN, it being also possible for one or more non-adjacent CH₂ groups to be replaced, in each case independently from one another, by -O-, -S-, -NH-, -NR⁰-, -SiR⁰R⁰⁰-, -CO-, -COO-, -OCO-, -OCO-O-, -S-CO-, -CO-S-, -CH=CH- or -C≡C- in such a manner that O and/or S atoms are not linked directly to one another,
- X: is -O-, -S-, -CO-, -COO-, -OCO-, -O-COO-, -CO-NR⁰-, -NR⁰-CO-, -OCH₂-, -CH₂O-, -SCH₂-, -CH₂S-, -CF₂O-, -OCF₂-, -CF₂S-, -SCF₂-, -CF₂CH₂-, -CH₂CF₂-, -CF₂CF₂-, -CH=N-, -N=CH-, -N=N-, -CH=CR⁰-, -CX¹=CX²-, -C=C-, -CH=CH-COO-, -OCO-CH=CH- or a single bond, and
- R⁰, R⁰⁰, X¹ and X²: have one of the meanings given above.

X is preferably -O-, -S-, -OCH₂-, -CH₂O-, -SCH₂-, -CH₂S-, -CF₂O-, -OCF₂-, -CF₂S-, -SCF₂-, -CH₂CH₂-, -CF₂CH₂-, -CH₂CF₂-, -CF₂CF₂-, -CH=N-, -N=CH-, -N=N-, -CH=CR⁰-, -CX¹=CX²-, -C≡C- or a single bond, in particular -O-, -S-, -C≡C-, -CX¹=CX²- or a single bond, very preferably a group that is able to from a conjugated system, such as -C≡C- or -CX¹=CX²-, or a single bond.

Typical groups Sp' are, for example, -(CH₂)ₚ-, -(CH₂CH₂O)_{q}-CH₂CH₂-, -CH₂CH₂-S-CH₂CH₂- or -CH₂CH₂-NH-CH₂CH₂- or -(SiR⁰R⁰⁰-O)ₚ-, with p being an integer from 2 to 12, q being an integer from 1 to 3 and R⁰ and R⁰⁰ having the meanings given above.

Preferred groups Sp' are ethylene, propylene, butylene, pentylene, hexylene, heptylene, octylene, nonylene, decylene, undecylene, dodecylene, octadecylene, ethyleneoxyethylene, methyleneoxybutylene, ethylene-thioethylene, ethylene-N-methyliminoethylene, 1-methylalkylene, ethenylene, propenylene and butenylene for example.

Further preferred are compounds with one or two groups P-Sp- wherein Sp is a single bond.

In case of compounds with two groups P-Sp, each of the two polymerisable groups P and the two spacer groups Sp can be identical or different.

SCLCPs obtained from the inventive compounds or mixtures by polymerisation or copolymerisation have a backbone that is formed by the polymerisable group P.

The mono-, oligo- and polymers of the present invention can be synthesized according to or in analogy to known methods. Some preferred methods are described below.

The key step to the synthesis of all polymers is the preparation of benzodithiophenes containing substituents, preferably alkyl chains, in the 4,8- or 3,7- positions. The preparation of 4,8-dialkyl or otherwise substituted monomer is depicted in Scheme 1 below, wherein R has one of the meanings of R¹.

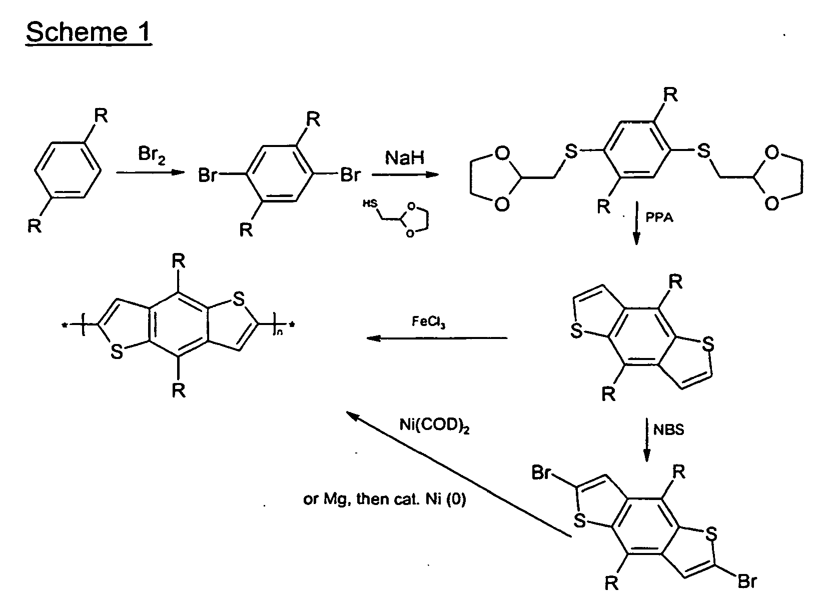

Firstly 1 ,4-dialkylbenzene is brominated in the 2,5- positions by treatment with bromine [see H. Kukula; S. Veit; A. Godt, Eur. J. Org. Chem, 1999, 277-286]. This is treated with two protected mercaptoethanal equivalents in the presence of base, and optionally a transition metal catalyst such as Pd(PPh₃)₄. Examples of protected 2-mercaptoethanals are 2-mercaptomethyl-1,3-dioxolane (HSCH₂CH(OCH₂CH₂O) and 2-mercapto-1,1-dimethoxyethane (HSCH₂CH(OCH₃)₂). Finally the protecting group is removed under acidic conditions and the thiophene ring formed by an acid catalysed Friedal-Crafts type intramolecular cyclisation and subsequent dehydration of the resulting alcohol in situ. This follows procedures similar to those reported for the preparation of 4,8-dimethylbenzodithiophene [see M. Pomerantz; J. Wang; S. Seong; K. P. Starkey; L. Nguyen; D. S. Marynick, Macromolecules, 1994, 27, 7478-7485 and P. Beimling; G. Kossmehl, Chem. Ber., 1986, 119, 3198-3203]. The resulting benzodithiophene can be polymerised directly by oxidative coupling with for example ferric (III) chloride. Alternatively the monomer can be brominated in the 2,6- positions and then chemically polymerised to the homopolymer by treatment with Ni(COD)₂ (Yamamoto coupling) [see K. Shiraishi; T. Yamamoto, Synthetic Metals, 2002, 130, 139-147], the dibromo monomer can also be polymerised by formation of a mono Grignard or organozinc reagent, and treatment of this intermediate with a nickel catalyst (McCullough type polymerisation) [see S. M. K. Robert S. Loewe, and; R. D. McCullough*, Adv. Mater., 1999, 11, 250].

The dibromo intermediate can also be used to form co-polymers by co-polymerisation with another suitable difunctionalised monomer, such as a bis(boronic) acid or ester, or a di-organotin reagent as demonstrated in Scheme 2, wherein R has one of the meanings of R¹ and Ar and n are as defined above.

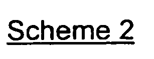

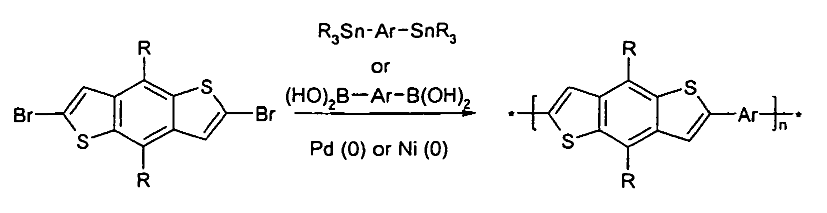

The preparation of 3,7-dialkyl benzodithiophene is depicted in Scheme 3 below, wherein R has one of the meanings of R¹, and follows a similar precedent to that described above.

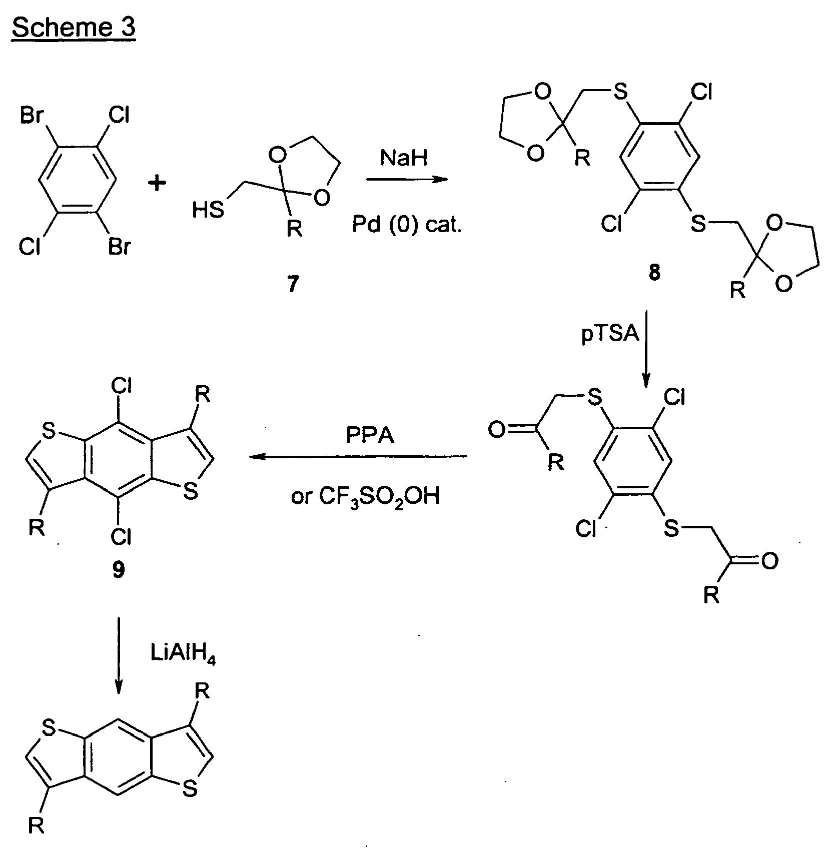

Firstly a ketone protected mercaptan such as **7** is prepared as described in the literature [see L. Novak; P. Kolonits; C. Szantay; J. Aszodi; M. Kajtar, Tetrahedron, 1982, 38, 153-159]. This is reacted with a 1,4-dibromo-2,5-dichlorobenzene under basic conditions in the presence of a transition metal catalyst to afford dichlorobenzene intermediate **8**. The ketal protecting groups are removed under acidic conditions, and the resulting ketone's are cyclised and dehydrated under acidic conditions to afford benzodithiophene intermediate **9** [see R. W. Guthrie; G. L. Kaplan; F. A. Mennona; J. W. Tilley; R. W. Kierstead; M. O'Donnell; H. Crowley; B. Yaremko; A. F. Welton, J. Med. Chem., 1990, 33, 2856-2864]. Finally the chlorine atoms, which are necessary to direct the regiochemistry of the cyclisation, are removed by treatment with lithium aluminium hydride. The resulting monomer can be polymerised as descibed above.

A further aspect of the invention relates to both the oxidised and reduced form of the compounds and materials according to this invention. Either loss or gain of electrons results in formation of a highly delocalised ionic form, which is of high conductivity. This can occur on exposure to common dopants. Suitable dopants and methods of doping are known to those skilled in the art, e.g., from EP 0 528 662, US 5,198,153 or WO 96/21659.

The doping process typically implies treatment of the semiconductor material with an oxidating or reducing agent in a redox reaction to form delocalised ionic centres in the material, with the corresponding counterions derived from the applied dopants. Suitable doping methods comprise for example exposure to a doping vapor in the atmospheric pressure or at a reduced pressure, electrochemical doping in a solution containing a dopant, bringing a dopant into contact with the semiconductor material to be thermally diffused, and ion-implantantion of the dopant into the semiconductor material.

When electrons are used as carriers, suitable dopants are for example halogens (e.g., l₂, Cl₂, Br₂, lCl, lCl₃, lBr and IF), Lewis acids (e.g., PF₅, AsF₅, SbF₅, BF₃, BCl₃, SbCl₅, BBr₃ and SO₃), protonic acids, organic acids, or amino acids (e.g., HF, HCl, HNO₃, H₂SO₄, HClO₄, FSO₃H and CISO₃H), transition metal compounds (e.g., FeCl₃, FeOCl, Fe(ClO₄)₃, Fe(4-CH₃C₆H₄SO₃)₃, TiCl₄, ZrCl₄, HfCl₄, NbF₅, NbCl₅, TaCl₅, MoF₅, MoCl₅, WF₅, WCl₆, UF₆ and LnCl₃ (wherein Ln is a lanthanoid), anions (e.g., Cl⁻, Br , I⁻, l₃⁻ , HSO₄⁻, SO₄²⁻, NO₃⁻, ClO₄⁻, BF₄⁻, PF₆⁻, AsF₆⁻, SbF₆⁻, FeCl₄⁻, Fe(CN)₆³⁻, and anions of various sulfonic acids, such as aryl-SO₃⁻). When holes are used as carriers, examples of dopants are cations (e.g., H⁺, Li⁺, Na⁺, K⁺, Rb⁺ and Cs⁺), alkali metals (e.g., Li, Na, K, Rb, and Cs), alkaline-earth metals (e.g., Ca, Sr, and Ba), O₂, XeOF₄, (NO₂⁺) (SbF₆⁻), (NO₂⁺) (SbCl₆⁻), (NO₂⁺) (BF₄⁻), AgClO₄, H₂lrCl₆, La(NO₃)₃ ^{·} 6H₂O, FSO₂OOSO₂F, Eu, acetylcholine, R₄N⁺, (R is an alkyl group), R₄P⁺ (R is an alkyl group), R₆As⁺ (R is an alkyl group), and R₃S⁺ (R is an alkyl group).

The conducting form of the compounds and materials of the present invention can be used as an organic "metal" in applications, for example, but not limited to, charge injection layers and ITO planarising layers in organic light emitting diode applications, films for flat panel displays and touch screens, antistatic films, printed conductive substrates, patterns or tracts in electronic applications such as printed circuit boards and condensers.

A preferred embodiment of the present invention relates to mono-, oligo- and polymers of formula I and its preferred subformulae that are mesogenic or liquid crystalline, and very preferably comprise one or more polymerisable groups. Very preferred materials of this type are monomers and oligomers of formula I and its preferred subformulae wherein n is an integer from 1 to 15 and R⁵ and/or R⁶ denote P-Sp-.

These materials are particularly useful as semiconductors or charge transport materials, as they can be aligned into uniform highly ordered orientation in their liquid crystal phase by known techniques, thus exhibiting a higher degree of order that leads to particularly high charge carrier mobility. The highly ordered liquid crystal state can be fixed by in situ polymerisation or crosslinking via the groups P to yield polymer films with high charge carrier mobility and high thermal, mechanical and chemical stability.

For example, if a device is made from a polymerisable liquid crystal material by polymerisation in situ, the liquid crystal material preferably comprises one or more mono- or oligomers of formula I and its preferred subformulae wherein one or both of R⁵ and R⁶ denote P-Sp-. If a liquid crystal polymer is preapred first, for example by polymerisation in solution, and the isolated polymer is used to make the device, the polymer is preferably made from a liquid crystal material comprising one or more mono- or oligomers of formula I and its preferred subformulae wherein one of R⁵ and R⁶ denotes P-Sp-.

It is also possible to copolymerise the polymerisable mono-, oligo- and polymers according to the present invention with other polymerisable mesogenic or liquid crystal monomers that are known from prior art, in order to induce or enhance liquid crystal phase behaviour.

Thus, another aspect of the invention relates to a polymerisable liquid crystal material comprising one or more mono-, oligo- or polymers of the present invention as described above and below comprising at least one polymerisable group, and optionally comprising one or more further polymerisable compounds, wherein at least one of the polymerisable mono-, oligo- and polymers of the present invention and/or the further polymerisable compounds is mesogenic or liquid crystalline.

Particularly preferred are liquid crystal materials having a nematic and/or smectic phase. For FET applications smectic materials are especially preferred. For OLED applications nematic or smectic materials are especially preferred.

Another aspect of the present invention relates to an anisotropic polymer film with charge transport properties obtainable from a polymerisable liquid crystal material as defined above that is aligned in its liquid crystal phase into macroscopically uniform orientation and polymerised or crosslinked to fix the oriented state.

Preferably polymerisation is carried out as in-situ polymerisation of a coated layer of the material, preferably during fabrication of the electronic or optical device comprising the inventive semiconductor material. In case of liquid crystal materials, these are preferably aligned in their liquid crystal state into homeotropic orientation prior to polymerisation, where the conjugated pi-electron systems are orthogonal to the direction of charge transport. This ensures that the intermolecular distances are minimised and hence then energy required to transport charge between molecules is minimised. The molecules are then polymerised or crosslinked to fix the uniform orientation of the liquid crystal state. Alignment and curing are carried out in the liquid crystal phase or mesophase of the material. This technique is known in the art and is generally described for example in D.J. Broer, et al., Angew. Makromol. Chem. 183, (1990), 45-66

Alignment of the liquid crystal material can be achieved for example by treatment of the substrate onto which the material is coated, by shearing the material during or after coating, by application of a magnetic or electric field to the coated material, or by the addition of surface-active compounds to the liquid crystal material. Reviews of alignment techniques are given for example by I. Sage in "Thermotropic Liquid Crystals", edited by G. W. Gray, John Wiley & Sons, 1987, pages 75-77, and by T. Uchida and H. Seki in "Liquid Crystals - Applications and Uses Vol. 3", edited by B. Bahadur, World Scientific Publishing, Singapore 1992, pages 1-63. A review of alignment materials and techniques is given by J. Cognard, Mol. Cryst. Liq. Cryst. 78, Supplement 1 (1981), pages 1-77.

Polymerisation takes place by exposure to heat or actinic radiation. Actinic radiation means irradiation with light, like UV light, IR light or visible light, irradiation with X-rays or gamma rays or irradiation with high energy particles, such as ions or electrons. Preferably polymerisation is carried out by UV irradiation at a non-absorbing wavelength. As a source for actinic radiation for example a single UV lamp or a set of UV lamps can be used. When using a high lamp power the curing time can be reduced. Another possible source for actinic radiation is a laser, like e.g. a UV laser, an IR laser or a visible laser.

Polymerisation is preferably carried out in the presence of an initiator absorbing at the wavelength of the actinic radiation. For example, when polymerising by means of UV light, a photoinitiator can be used that decomposes under UV irradiation to produce free radicals or ions that start the polymerisation reaction. When curing polymerisable materials with acrylate or methacrylate groups, preferably a radical photoinitiator is used, when curing polymerisable materials with vinyl, epoxide and oxetane groups, preferably a cationic photoinitiator is used. It is also possible to use a polymerisation initiator that decomposes when heated to produce free radicals or ions that start the polymerisation. As a photoinitiator for radical polymerisation for example the commercially available Irgacure 651, Irgacure 184, Darocure 1173 or Darocure 4205 (all from Ciba Geigy AG) can be used, whereas in case of cationic photopolymerisation the commercially available UVI 6974 (Union Carbide) can be used.

The polymerisable material can additionally comprise one or more other suitable components such as, for example, catalysts, sensitizers, stabilizers, inhibitors, chain-transfer agents, co-reacting monomers, surface-active compounds, lubricating agents, wetting agents, dispersing agents, hydrophobing agents, adhesive agents, flow improvers, defoaming agents, deaerators, diluents, reactive diluents, auxiliaries, colourants, dyes or pigments.

Mono-, oligo- and polymers comprising one or more groups P-Sp- can also be copolymerised with polymerisable mesogenic compounds to induce or enhance liquid crystal phase behaviour. Polymerisable mesogenic compounds that are suitable as comonomers are known in prior art and disclosed for example in WO 93/22397; EP 0,261,712; DE 195,04,224; WO 95/22586 and WO 97/00600.

Another aspect of the invention relates to a liquid crystal side chain polymer (SCLCP) obtained from a polymerisable liquid crystal material as defined above by polymerisation or polymeranaloguous reaction. Particularly preferred are SCLCPs obtained from one or more monomers of formula 11 and its preferred subformulae wherein one or both, preferably one, of R⁵ and R⁶ are a polymerisable or reactive group, or from a polymerisable mixture comprising one or more of said monomers.

Another aspect of the invention relates to an SCLCP obtained from one or more monomers of formula I1 and its preferred subformulae wherein one or both of R⁵ and R⁶ are a polymerisable group, or from a polymerisable liquid crystal mixture as defined above, by copolymerisation or polymeranaloguous reaction together with one or more additional mesogenic or non-mesogenic comonomers.

Side chain liquid crystal polymers or copolymers (SCLCPs), in which the semiconducting component is located as a pendant group, separated from a flexible backbone by an aliphatic spacer group, offer the possibility to obtain a highly ordered lamellar like morphology. This structure consists of closely packed conjugated aromatic mesogens, in which very close (typically < 4 Å) pi-pi stacking can occur. This stacking allows intermolecular charge transport to occur more easily, leading to high charge carrier mobilities. SCLCPs are advantageous for specific applications as they can be readily synthesized before processing and then e.g. be processed from solution in an organic solvent. If SCLCPs are used in solutions, they can orient spontaneously when coated onto an appropriate surface and when at their mesophase temperature, which can result in large area, highly ordered domains.

SCLCPs can be prepared from the polymerisable compounds or mixtures according to the invention by the methods described above, or by conventional polymerisation techniques which are known to those skilled in the art, including for example radicalic, anionic or cationic chain polymerisation, polyaddition or polycondensation. Polymerisation can be carried out for example as polymerisation in solution, without the need of coating and prior alignment, or polymerisation in situ. It is also possible to form SCLCPs by grafting compounds according to the invention with a suitable reactive group, or mixtures thereof, to presynthesized isotropic or anisotropic polymer backbones in a polymeranaloguous reaction. For example, compounds with a terminal hydroxy group can be attached to polymer backbones with lateral carboxylic acid or ester groups, compounds with terminal isocyanate groups can be added to backbones with free hydroxy groups, compounds with terminal vinyl or vinyloxy groups can be added, e.g., to polysiloxane backbones with Si-H groups. It is also possible to form SCLCPs by copolymerisation or polymeranaloguous reaction from the inventive compounds together with conventional mesogenic or non mesogenic comonomers. Suitable comonomers are known to those skilled in the art. In principle it is possible to use all conventional comonomers known in the art that carry a reactive or polymerisable group capable of undergoing the desired polymer-forming reaction, like for example a polymerisable or reactive group P as defined above. Typical mesogenic comonomers are for example those mentioned in WO 93/22397, EP 0 261 712, DE 195 04 224, WO 95/22586, WO 97/00600 and GB 2 351 734. Typical non mesogenic comonomers are for example alkyl acrylates or alkyl methacrylates with alkyl groups of 1 to 20 C atoms, like methyl acrylate or methyl methacrylate.

The mono-, oligo- and polymers of the present invention are useful as optical, electronic and semiconductor materials, in particular as charge transport materials in field effect transistors (FETs), e.g., as components of integrated circuitry, ID tags or TFT applications. Alternatively, they may be used in organic light emitting diodes (OLEDs) in electroluminescent display applications or as backlight of, e.g., liquid crystal displays, as photovoltaics or sensor materials, for electrophotographic recording, and for other semiconductor applications.

Especially the oligomers and polymers according to the invention show advantageous solubility properties which allow production processes using solutions of these compounds. Thus films, including layers and coatings, may be generated by low cost production techniques, e.g., spin coating. Suitable solvents or solvent mixtures comprise alkanes and/ or aromatics, especially their fluorinated derivatives.

The materials of the present invention are useful as optical, electronic and semiconductor materials, in particular as charge transport materials in field effect transistors (FETs), as photovoltaics or sensor materials, for electrophotographic recording, and for other semiconductor applications. Such FETs, where an organic semiconductive material is arranged as a film between a gate-dielectric and a drain and a source electrode, are generally known, e.g., from US 5,892,244, WO 00/79617, US 5,998,804, and from the references cited in the background and prior art chapter and listed below. Due to the advantages, like low cost production using the solubility properties of the compounds according to the invention and thus the processibility of large surfaces, preferred applications of these FETs are such as integrated circuitry, TFT-displays and security applications.

In security applications, field effect transistors and other devices with semiconductive materials, like transistors or diodes, may be used for ID tags or security markings to authenticate and prevent counterfeiting of documents of value like banknotes, credit cards or ID cards, national ID documents, licenses or any product with monetry value, like stamps, tickets, shares, cheques etc..

Alternatively, the mono-, oligo- and polymers according to the invention may be used in organic light emitting devices or diodes (OLEDs), e.g., in display applications or as backlight of e.g. liquid crystal displays. Common OLEDs are realized using multilayer structures. An emission layer is generally sandwiched between one or more electron-transport and/ or hole-transport layers. By applying an electric voltage electrons and holes as charge carriers move towards the emission layer where their recombination leads to the excitation and hence luminescence of the lumophor units contained in the emission layer. The inventive compounds, materials and films may be employed in one or more of the charge transport layers and/or in the emission layer, corresponding to their electrical and/ or optical properties. Furthermore their use within the emission layer is especially advantageous, if the compounds, materials and films according to the invention show electroluminescent properties themselves or comprise electroluminescent groups or compounds. The selection, characterization as well as the processing of suitable monomeric, oligomeric and polymeric compounds or materials for the use in OLEDs is generally known by a person skilled in the art, see, e.g., Meerholz, Synthetic Materials, 111-112, 2000, 31-34, Alcala, J. Appl. Phys., 88, 2000, 7124-7128 and the literature cited therein.

According to another use, the inventive compounds, materials or films, especially those which show photoluminescent properties, may be employed as materials of light sources, e.g., of display devices such as described in EP 0 889 350 A1 or by C. Weder et al., Science, 279, 1998, 835-837.

According to another use, the inventive compounds, materials or films can be used alone or together with other materials in or as alignment layers in LCD or OLED devices, as described for example in US 2003/0021913. The use of charge transport compounds according to the present invention can increase the electrical conductivity of the alignment layer. When used in an LCD, this increased electrical conductivity can reduce adverse residual dc effects in the switchable LCD cell and suppress image sticking or, for example in ferroelectric LCDs, reduce the residual charge produced by the switching of the spontaneous polarisation charge of the ferroelectric LCs. When used in an OLED device comprising a light emitting material provided onto the alignment layer, this increased electrical conductivity can enhance the electroluminescence of the light emitting material. The compounds or materials according to the present invention having mesogenic or liquid crystalline properties can form oriented anisotropic films as described above, which are especially useful as alignment layers to induce or enhance alignment in a liquid crystal medium provided onto said anisotropic film. The materials according to the present invention may also be combined with photoisomerisable compounds and/or chromophores for use in or as photoalignment layers, as described in US 2003/0021913.

According to another use the polymers according to the present invention, especially their water-soluble derivatives (for example with polar or ionic side groups) or ionically doped forms, can be employed as chemical sensors or materials for detecting and discriminating DNA sequences. Such uses are described for example in L. Chen, D. W. McBranch, H. Wang, R. Helgeson, F. Wudl and D. G. Whitten, Proc. Natl. Acad. Sci. U.S.A. 1999, 96, 12287; D. Wang, X. Gong, P. S. Heeger, F. Rininsland, G. C. Bazan and A. J. Heeger, Proc. Natl. Acad. Sci. U.S.A. 2002, 99, 49; N. DiCesare, M. R. Pinot, K. S. Schanze and J. R. Lakowicz, Langmuir 2002, 18, 7785; D. T. McQuade, A. E. Pullen, T. M. Swager, Chem. Rev. 2000, 100, 2537.

The examples below serve to illustrate the invention without limiting it. In the foregoing and the following, all temperatures are given in degrees Celsius, and all percentages are by weight, unless stated otherwise.

### Example 1

Poly-(4,8-dihexylbenzo[1,2-b;4,5-b']dithiophene) (1d) is prepared as shown in Scheme 1 above.

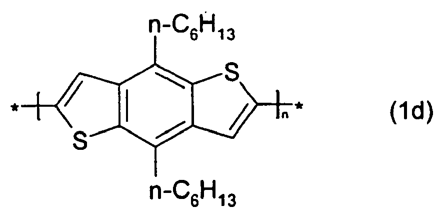

### 1a) Sodium [1,3]dioxolan-2-ylmethanethiolate

2-Bromomethyl[1,3]dioxolane (101.2 g, 0.606 mol) and potassium thioacetate (70.8 g, 0.620 mol) in DMF (700 mL) are stirred at 40°C for 70 h under nitrogen. The reaction mixture is cooled to RT and filtered. The solvent is removed under reduced pressure. The residue is dissolved in DCM (400 mL) and washed with water (400 mL). The aqueous layer is extracted again with DCM (2 x 100 mL). The combined extracts are washed with brine (2 x 100 mL), dried over sodium sulfate, and concentrated *in vacuo.* The residue is filtered through silica (eluent: DCM) and concentrated *in vacuo* to afford_thioacetic acid *S*-[1,3]dioxolan-2-ylmethyl ester (97.1 g, 99%). Thioacetic acid *S*-[1,3]dioxolan-2-ylmethyl ester (63.4 g, 0.391 mol) and sodium methoxide (21.6 g, 0.400 mol) in anhydrous methanol (1 L) are stirred at RT for 12 h. The solvent is removed under reduced pressure to yield the product as a yellow solid (37.5 g, 67%): ¹H NMR as expected.

### 1b) 2,5-Bis([1,3]dioxolan-2-ylmethanethiolato)-1,4-dihexylbenzene

A 3-necked flask is charged with sodium [1,3]dioxolan-2-ylmethanethiolate (1.78 g, 12.50 mmol) and sodium *tert*-butoxide (1.20 g, 12.5 mmol) and purged with nitrogen. Anhydrous toluene (30 mL) is added and the reaction mixture is stirred at RT overnight. Palladium(II) acetate (0.11 g, 0.50 mmol), *rac*-BINAP (0.34 g, 0.55 mmol) and 2,5-dibromo-1,4-dihexylbenzene (2.02 g, 5.00 mmol) are added and the reaction mixture is stirred at 80°C for 24 h. The reaction mixture is cooled to RT and diluted with EtOAc (100 mL). Water (100 mL) is added. The organic extract is collected. The aqueous layer is extracted again with EtOAc (100 mL). The combined extracts are washed with water (2 x 100 mL), dried over sodium sulfate, and concentrated *in vacuo.* Column chromatography (eluent: 50% DCM/ 50% petrol) yielded the product as a pale yellow oil (0.90 g, 37%): ¹H NMR (CDCl₃, 300 MHz): δ 7.18 (s, 2H, Ar*H*), 5.08 (t, ³*J* = 4.5 Hz, 2H, OC*H*O), 4.03 (m, 4H, OC*H*₂), 3.89 (m, 4H, OC*H*₂), 3.08 (d, ³*J* = 4.5 Hz, 4H, SC*H*₂), 2.70 (t, ³*J* = 8.0 Hz, 4H, ArC*H*₂), 1.57 (m, 4H, C*H*₂), 1.29 (br, 12H, C*H*₂), 0.88 (t, ³*J* = 6.5 Hz, 2H, C*H*₃).

### 1c) 4,8-Dihexylbenzo[1,2-b;4,5-b']dithiophene

A solution of 2,5-bis([1,3]dioxolan-2-ylmethanethiolato)-1,4-dihexylbenzene (0.48 g, 1.00 mmol) in chlorobenzene (5 mL) is added dropwise to a refluxing solution of 84% polyphosphoric acid (0.5 mL) in chlorobenzene (5 mL) under nitrogen. The reaction mixture is stirred at reflux for 60 h. After cooling to RT, the reaction mixture is poured into water (100 mL) and extracted into DCM (2 x 100 mL). The combined extracts are washed with water (100 mL), dried over sodium sulfate and concentrated *in vacuo.* Column chromatography (eluent: petroleum ether 40-60) yielded the product as a pale yellow solid (0.25 g, 69%): ¹H NMR (CDCl₃, 300 MHz): δ 7.48 (d, ³*J* = 5.5 Hz, 2H, Ar*H*), 7.45 (d, ³*J* = 5.5 Hz, 2H, Ar*H*), 3.18 (t, ³*J* = 8.0 Hz, 4H, ArC*H*₂), 1.81 (m, 4H, C*H*₂), 1.48 (m, 4H, C*H*₂), 1.34 (br, 8H, C*H*₂), 0.90 (t, ³*J* = 6.5 Hz, 6H, C*H*₃).

### 1d) Poly(4,8-dihexylbenzo[1,2-b;4,5-b']dithiophene)

A solution of 4,8-dihexylbenzo[1,2-b;4,5-b']dithiophene (0.20 g, 0.55 mmol) in anhydrous CHCl₃ (10 mL) is added to a stirred solution of anhydrous FeCl₃ (0.45 g, 2.75 mmol) in anhydrous CHCl₃ (40 mL) in a 3-necked flask fitted with a condenser. The reaction mixture is stirred for 20 h at RT with a constant flow of nitrogen bubbled into the reaction mixture to remove HCl formed. The reaction mixture is poured into methanol (300 mL) and stirred for 1 h. The polymer is collected by filtration and washed with water followed by methanol. The polymer is de-doped by stirring in conc. NH₄OH (50 mL, 33%) for 1 h. The precipitate is collected by filtration, washed with water followed by methanol, and dried under vacuum. The polymer is washed (*via* Soxhlet extraction) with methanol for 21 h and petroleum ether 40-60 for 5 h. before being dried under vacuum to yield the product as a dark red solid (0.15 g, 75%).

### Example 2

Poly-(4,8-didecylbenzo[1,2-b;4,5-b']dithiophene) (2d) is prepared as shown in Scheme 1 above.

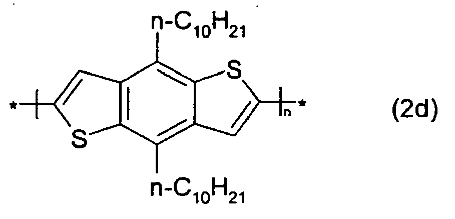

### 2a) 2,5-Dibromo-1,4-didecylbenzene

2,5-Dibromo-1,4-didecylbenzene is prepared from 1,4-dibromobenzene in two steps according to literature procedures: decylmagnesium bromide is coupled to 1,4-dibromobenzene using PdCl₂(dppf) to yield 1,4-didecylbenzene, which is subsequently treated with bromine to afford 2,5-dibromo-1,4-didecylbenzene.

### 2b) 2,5-Bis([1,3]dioxolan-2-ylmethanethiolato)-1,4-didecylbenzene

A 3-necked flask is charged with sodium [1,3]dioxolan-2-ylmethanethiolate (2.84 g, 20.00 mmol) and sodium *tert*-butoxide (2.31 g, 20.00 mmol) and purged with nitrogen. Anhydrous toluene (50 mL) is added and the reaction mixture is stirred at RT overnight. Palladium (II) acetate (0.18 g, 0.80 mmol), *rac*-BINAP (0.55 g, 0.88 mmol) and 2,5-dibromo-1,4-didecylbenzene (4.13 g, 8.00 mmol) are added and the reaction mixture is stirred at 80°C for 48 h. The reaction mixture is cooled to RT and diluted with EtOAc (200 mL). Water (100 mL) is added. The organic extract is collected. The aqueous layer is extracted again with EtOAc (200 mL). The combined extracts are washed with water (2 x 100 mL), dried over sodium sulfate, and concentrated *in vacuo.* Column chromatography (eluent: 50% DCM/ 50% petrol) yielded product as a pale yellow oil (3.20 g, 67%): ¹H NMR as expected.

### 2c) 4,8-Didecylbenzo[1,2-b;4,5-b']dithiophene

A solution of 2,5-bis([1,3]dioxolan-2-ylmethanethiolato)-1,4-didecylbenzene (3.00 g, 5.04 mmol) in chlorobenzene (25 mL) is added dropwise to a refluxing solution of 84% polyphosphoric acid (3 mL) in chlorobenzene (25 mL) under nitrogen. The reaction mixture is stirred at reflux for 7 days. After cooling to RT, the reaction mixture is poured into water (200 mL) and extracted into DCM (2 x 200 mL). The combined extracts are washed with water (100 mL), dried over sodium sulfate and concentrated *in vacuo.* Column chromatography (eluent: petroleum ether 40-60) yielded the product as a pale yellow solid (0.60 g, 25%): ¹H NMR as expected.

### 2d) Poly(4,8-didecylbenzo[1,2-b;4,5-b']dithiophene) via oxidative polymerisation

A solution of 4,8-didecylbenzo[1,2-b;4,5-b']dithiophene (0.30 g, 0.64 mmol) in anhydrous CHCl₃ (10 mL) is added to a stirred solution of anhydrous FeCl₃ (0.60 g, 3.69 mmol) in anhydrous CHCl₃ (40 mL) in a 3-necked flask fitted with a condenser. The reaction mixture is stirred for 48 h at RT with a constant flow of nitrogen bubbled into the reaction mixture to remove HCl formed. The reaction mixture is poured into methanol (300 mL) and stirred for 1 h. The polymer is collected by filtration and washed with water followed by methanol. The polymer is de-doped by stirring in conc. NH₄OH (50 mL, 33%) for 1 h. The precipitate is collected by filtration, washed with water followed by methanol, and dried under vacuum. The polymer is washed (*via* Soxhlet extraction) with methanol for 18 h and petroleum ether 40-60 for 6 h. before being dried under vacuum to yield the product as a dark red solid (0.21 g, 70%).

### 2e) 2,6-Dibromo-4,8-didecylbenzo[1,2-b;4,5-b']dithiophene

NBS (0,89 g, 5.00 mmol) is added to a solution of 4,8-didecylbenzo[1,2-b;4,5-b']dithiophene (1.16 g, 2.46 mmol) in CHCl₃ (10 mL) and AcOH (10 mL) under nitrogen. The reaction mixture is stirred at RT for 27 h. The reaction mixture is poured into water (50 mL) and extracted into DCM (2 x 100 mL). The combined extracts are washed with water (50 mL), dried over sodium sulfate, and concentrated *in vacuo.* Recrystallisation twice from acetone afforded the product as pale yellow needles (0.80 g, 53%):¹H NMR (CDCl₃, 300 MHz): δ 7.41 (s, 2H, Ar*H*), 2.98 (t, ³*J* = 8.0 Hz, 4H, ArC*H*₂), 1.73 (m, 4H, C*H*₂), 1.33 (br, 28H, C*H*₂), 0.88 (t, ³*J* = 6.5 Hz, 6H, C*H*₃).

### 2d) Poly(4,8-didecylbenzo[1,2-b:4.5-b']dithiophene) via Yamamoto coupling

A schlenk tube is charged with Ni(cod)₂ (0.26 g, 0.93 mmol) and bipy (0.15 g, 0.93 mmol) under nitrogen. cod (0.10 mL, 0.80 mmol) and anhydrous DMF (3 mL) are added. The reaction mixture is stirred at 60°C for 30 minutes. A solution of 2,6-dibromo-4,8-didecylbenzo[1,2-b;4,5-b']dithiophene (0.42 g, 0.66 mmol) in anhydrous toluene (9 mL) is added. The reaction mixture is stirred at 80°C for 24 h. Bromobenzene (0.2 mL) is added and the reaction mixture is stirred at 80°C for 1 h. After cooling to RT, the reaction mixture is precipitated into acidic methanol (300 mL) and stirred for 0.5 h. The precipitate is collected by filtration, washed with methanol, and dried under vacuum. The polymer is washed (*via* Soxhlet extraction) with methanol for 20 h and petroleum ether 40-60 for 6 h. before being dried under vacuum to yield the product as a dark red solid (0.33 g, 96%).

## Claims

1. Oligo- or polymers comprising one or more identical or different recurring units of formula I 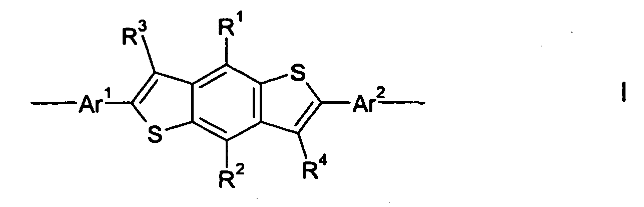 wherein
R¹ to R⁴ are independently of each other H, halogen or straight chain, branched or cyclic alkyl with 1 to 20 C-atoms, which may be unsubstituted, mono- or poly-substituted by F, Cl, Br, I or CN, it being also possible for one or more non-adjacent CH₂ groups to be replaced, in each case independently from one another, by -O-, -S-, -NH-, -NR⁰-, -SiR⁰R⁰⁰-, -CO-, - COO-, -OCO-, -OCO-O-, -S-CO-, -CO-S-, -CH=CH-or -C≡C- in such a manner that O and/or S atoms are not linked directly to one another, or optionally substituted aryl or heteroaryl, or P-Sp-,
P is a polymerisable or reactive group,
Sp is a spacer group or a single bond,
R⁰ and R⁰⁰ are independently of each other H or alkyl with 1 to 12 C-atoms,
Ar¹ and Ar² are independently of each other -CX¹=CX²-, -C≡C-, an arylene or heteroarylene group that is optionally substituted with one or more groups R¹, or a single bond,
X¹ and X² are independently of each other H, F, Cl or CN,
with the provisos that
a) in at least one recurring unit R¹ and R² are different from H and/or R³ and R⁴ are different from H,
b) R¹ and R² do not at the same time denote an alkoxy group,
and wherein the number of recurring units is from 10 to 5000.

2. Oligo- or polymers according to claim 1, selected of formula I1 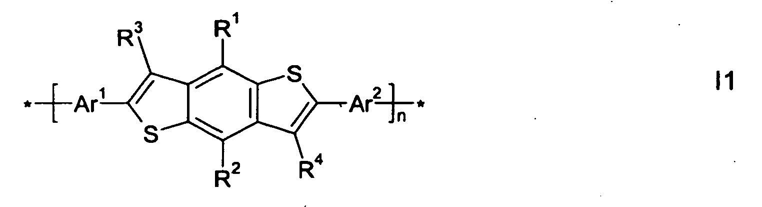 wherein R¹, R², R³, R⁴, Ar¹ and Ar² have independently of each other one of the meanings of claim 1, n is an integer from 10 to 5000, and wherein the recurring units may be identical or different.

3. Oligo- or polymers according to claim 2, selected of formula I1a 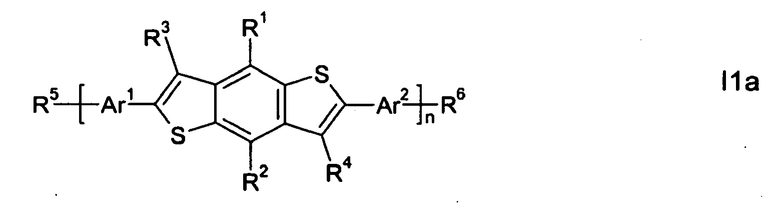 wherein R¹, R², R³, R⁴, Ar¹, Ar² and n have independently of each other one of the meanings of claim 2,
R⁵ and R⁶ are independently of each other H, halogen, - Sn(R⁰)₃, -B(OR')(OR"), -CH₂Cl, -CHO, -CH=CH₂,-SiR⁰R⁰⁰R⁰⁰⁰, optionally substituted aryl or heteroaryl or P-Sp-,
R⁰⁰⁰ has one of the meanings of R⁰ in claim 1,
R' and R" are independently of each other H or alkyl with 1 to 12 C-atoms, or OR' and OR" together with the boron atom form a cyclic group having 2 to 20 C atoms, and
P, Sp, R⁰ and R⁰⁰ have the meanings of claim 1.

4. Monomers of formula I1a 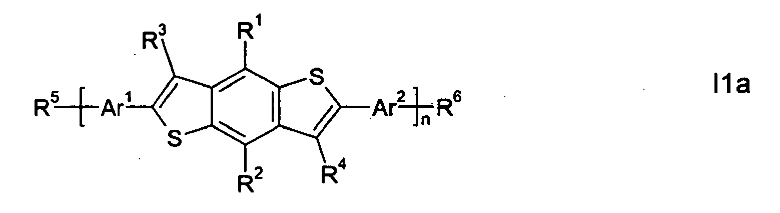 wherein R¹, R², R³, R⁴, Ar¹ and Ar² have independently of each other one of the meanings of claim 2, n is 1, and R⁵ and R⁶ are selected from Cl, Br, I, -Sn(R⁰)₃, -B(OR')(OR"), -CH₂Cl, -CHO and -SiR⁰R⁰⁰R⁰⁰⁰_{.}

5. Mono-, oligo- or polymers according to at least one of claims 1 to 4, wherein R¹ and R² are selected from C₁-C₂₀-alkyl that is optionally substituted with one or more fluorine atoms, C₁-C₂₀-alkenyl, C₁-C₂₀-alkynyl, C₁-C₂₀-thioether, C₁-C₂₀-silyl, C₁-C₂₀-ester, C₁-C₂₀-amino, C₁-C₂₀-fluoroalkyl and optionally substituted aryl or heteroaryl, and R³ and R⁴ are H.

6. Mono-, oligo- or polymers according to at least one of claims 1 to 5, wherein R³ and R⁴ are selected from C₁-C₂₀-alkyl that is optionally substituted with one or more fluorine atoms, C₁-C₂₀-alkenyl, C₁-C₂₀-alkynyl, C₁-C₂₀-thioether, C₁-C₂₀-silyl, C₁-C₂₀-ester, C₁-C₂₀-amino, C₁-C₂₀-fluoroalkyl and optionally substituted aryl or heteroaryl, and R¹ and R² are H.

7. Mono-, oligo- or polymers according to at least one of claims 1 to 7, wherein Ar¹ and/or Ar² are selected from phenyl in which, in addition, one or more CH groups may be replaced by N, or naphthalene, alkyl fluorene or oxazole, wherein these groups are optionally mono- or polysubstituted with L, wherein L is F, Cl, Br, or an alkyl, alkoxy, alkylcarbonyl, alkylcarbonyloxy or alkoxycarbonyl group with 1 to 12 C atoms, wherein one or more H atoms are optionally replaced by F or Cl, or thiophene, thienothiophene or dithienothiophene which are substituted by one or more halogen atoms.

8. Mono-, oligo- or polymers according to at least one of claims 1 to 7, wherein Ar¹ and/or Ar² are selected from 1 ,4-phenylene, fluorinated 1,4-phenylene, 2,5-pyridine, 2,5-pyrimidine, p,p'-biphenyl, naphthalene-2,6-diyl, thiophene-2,5-diyl, fluorinated or alkylated thiophene-2,5-diyl, fluorinated benzo[1,2-b:4,5-b']dithiophene, 2,5-thiazole, 2,5-thiadiazole, 2,5-oxazole and 2,5-oxadiazole, all of which are unsubstituted, mono- or polysubstituted with L, wherein L is F, Cl, Br, or an alkyl, alkoxy, alkylcarbonyl, alkylcarbonyloxy or alkoxycarbonyl group with 1 to 12 C atoms, wherein one or more H atoms are optionally replaced by F or Cl.

9. Mono-, oligo- or polymers according to claim 8, wherein Ar¹ and Ar² are thiophene-2,5-diyl that is optionally substituted.

10. Mono-, oligo- or polymers according to at least one of claims 1 to 9, wherein Ar¹ and/or Ar² are a single bond.

11. Polymerisable liquid crystal material comprising one or more mono-, oligo- or polymers according to at least one of the preceding claims comprising at least one polymerisable group, and optionally comprising one or more further polymerisable compounds, wherein at least one of said mono-, oligo- or polymers or further polymerisable compounds is mesogenic or liquid crystalline.

12. Anisotropic polymer film with charge transport properties obtainable from a polymerisable liquid crystal material according to claim 11 that is aligned in its liquid crystal phase into macroscopically uniform orientation and polymerised or crosslinked to fix the oriented state.

13. Side chain liquid crystal polymer obtained by polymerisation of one or more mono- or oligomers or a polymerisable material according to at least one of the preceding claims or by grafting one or more mono- or oligomers or a polymerisable material according to at least one of the preceding claims to a polymer backbone in a polymeranaloguous reaction, optionally with one or more additional mesogenic or non-mesogenic comonomers.

14. Use of the mono-, oligo- and polymers, polymerisable materials and polymers according to at least one of the preceding claims as semiconductors or charge transport materials in optical, electrooptical or electronic devices, like field effect transistors (FET) for example as components of integrated circuitry, of thin film transistors (TFT) for flat panel display applications, or of radio frequency identification (RFID) tags, or in semiconducting components for displays or organic light emitting diode (OLED) applications including both the charge transport and electroluminescent layer.

15. Use of the mono-, oligo- and polymers, polymerisable materials and polymers according to at least one of the preceding claims as electroluminescent materials, for OLED applications such as electroluminescent displays or backlights of displays, in photovoltaic or sensor devices, as electrode materials in batteries, as photoconductors, for electrophotographic applications like electrophotographic recording, for organic memory devices, for detecting and discriminating DNA sequences, and as alignment layer in LCD or OLED devices.

16. Optical, electrooptical or electronic device, FET, integrated circuit (IC), TFT, OLED or alignment layer comprising a material, component or device according to at least one of the preceding claims.

17. TFT or TFT array for flat panel displays, radio frequency identification (RFID) tag, electroluminescent display or backlight, comprising a material, component or device according to at least one of the preceding claims or a FET, IC, TFT or OLED according to claim 16.

18. Security marking or device comprising a FET or an RFID tag according to claim 17.

19. Mono-, oligo- and polymer, material or polymer according to at least one of the preceding claims, which is oxidatively or reductively doped to form conducting ionic species.

20. Charge injection layer, planarising layer, antistatic film or conducting substrate or pattern for electronic applications or flat panel displays, comprising a mono-, oligo- or polymer, material or polymer according to claim 19.

## Patentansprüche

1. Oligo- oder Polymere enthaltend eine oder mehrere gleiche oder verschiedene wiederkehrende Einheiten der Formel I 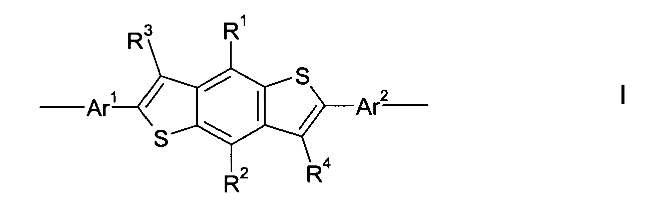 worin
R¹ bis R⁴ unabhängig voneinander H, Halogen oder geradkettiges, verzweigtes oder cyclisches Alkyl mit 1 bis 20 C-Atomen bedeuten, das unsubstituiert oder ein- oder mehrfach durch F, Cl, Br, I oder CN substituiert sein kann, wobei auch eine oder mehrere nicht benachbarte CH₂-Gruppen jeweils unabhängig voneinander so durch -O-, -S-, -NH-, -NR⁰-, -SiR⁰R⁰⁰-, -CO-, -COO-, -OCO-, -OCO-O-, -S-CO-, -CO-S-, -CH=CH- oder -C≡C- ersetzt sein können, dass O-und/oder S-Atome nicht direkt miteinander verknüpft sind, oder gegebenenfalls substituiertes Aryl oder Heteroaryl bedeuten oder für P-Sp- stehen,
P eine polymerisierbare oder reaktive Gruppe bedeutet,
Sp eine Spacergruppe oder eine Einfachbindung bedeutet,
R⁰ und R⁰⁰ unabhängig voneinander H oder Alkyl mit 1 bis 12 C-Atomen bedeuten,
Ar¹ und Ar² unabhängig voneinander -CX¹=CX²-, -C≡C-, eine Arylen- oder Heteroarylengruppe, die gegebenenfalls mit einer oder mehreren Gruppen R¹ substituiert ist, oder eine Einfachbindung bedeuten,
X¹ und X² unabhängig voneinander H, F, Cl oder CN bedeuten,
mit den Maßgaben, dass
a) in mindestens einer wiederkehrenden Einheit R¹ und R² von H verschieden sind und/oder R³ und R⁴ von H verschieden sind,
b) R¹ und R² nicht gleichzeitig eine Alkoxygruppe bedeuten,
und worin die Anzahl der wiederkehrenden Einheiten 10 bis 5000 beträgt.

2. Oligo- oder Polymere nach Anspruch 1, ausgewählt aus der Formel I1 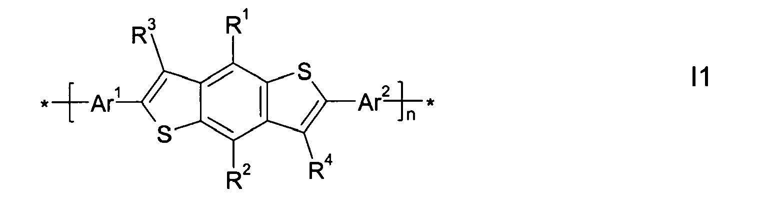 worin R¹, R², R³, R⁴, Ar¹ und Ar² unabhängig voneinander eine der Bedeutungen des Anspruchs 1 besitzen, n für eine ganze Zahl von 10 bis 5000 steht und worin die wiederkehrenden Einheiten gleich oder verschieden sein können.

3. Oligo- oder Polymere nach Anspruch 2, ausgewählt aus der Formel I1a 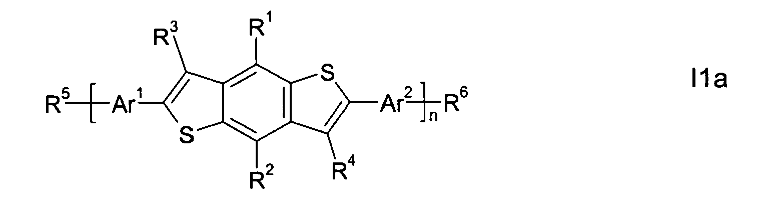 worin R¹, R², R³, R⁴, Ar¹, Ar² und n unabhängig voneinander eine der Bedeutungen des Anspruchs 2 besitzen,
R⁵ und R⁶ unabhängig voneinander H, Halogen, -Sn(R⁰)₃, -B(OR')(OR"), -CH₂Cl, -CHO, -CH=CH₂, -SiR⁰R⁰⁰R⁰⁰⁰, gegebenenfalls substituiertes Aryl oder Heteroaryl oder P-Sp- bedeuten,
R⁰⁰⁰ eine der Bedeutungen von R⁰ in Anspruch 1 besitzt,
R' und R" unabhängig voneinander H oder Alkyl mit 1 bis 12 C-Atomen bedeuten, oder OR' und OR" zusammen mit dem Boratom eine cyclische Gruppe mit 2 bis 20 C-Atomen bilden, und
P, Sp, R⁰ und R⁰⁰ die Bedeutungen des Anspruchs 1 besitzen.

4. Monomere der Formel I1a 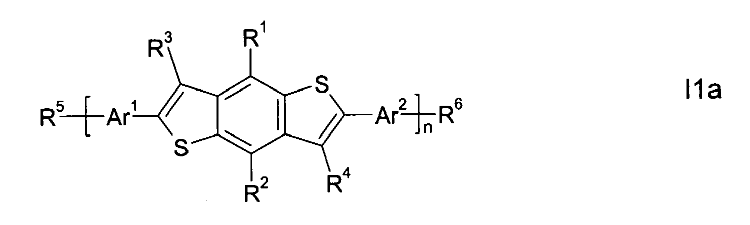 worin R¹, R², R³, R⁴, Ar¹ und Ar² unabhängig voneinander eine der Bedeutungen des Anspruchs 2 besitzen, n für 1 steht und R⁵ und R⁶ ausgewählt sind aus Cl, Br, I, -Sn(R⁰)₃, -B(OR')(OR"), -CH₂Cl, -CHO und -SiR⁰R⁰⁰R⁰⁰⁰.

5. Mono-, Oligo- oder Polymere nach mindestens einem der Ansprüche 1 bis 4, worin R¹ und R² ausgewählt sind aus C₁-C₂₀-Alkyl, das gegebenenfalls mit einem oder mehreren Fluoratomen substituiert ist, C₁-C₂₀-Alkenyl, C₁-C₂₀-Alkinyl, C₁-C₂₀-Thioether, C₁-C₂₀-Silyl, C₁-C₂₀-Ester, C₁-C₂₀-Amino, C₁-C₂₀-Fluoralkyl und gegebenenfalls substituiertem Aryl oder Heteroaryl, und R³ und R⁴ H bedeuten.

6. Mono-, Oligo- oder Polymere nach mindestens einem der Ansprüche 1 bis 5, worin R³ und R⁴ ausgewählt sind aus C₁-C₂₀-Alkyl, das gegebenenfalls mit einem oder mehreren Fluoratomen substituiert ist, C₁-C₂₀-Alkenyl, C₁-C₂₀-Alkinyl, C₁-C₂₀-Thioether, C₁-C₂₀-Silyl, C₁-C₂₀-Ester, C₁-C₂₀-Amino, C₁-C₂₀-Fluoralkyl und gegebenenfalls substituiertem Aryl oder Heteroaryl und R¹ und R² H bedeuten.

7. Mono-, Oligo- oder Polymere nach mindestens einem der Ansprüche 1 bis 7, worin Ar¹ und/oder Ar² ausgewählt sind aus Phenyl, in dem zusätzlich eine oder mehrere CH-Gruppen durch N ersetzt sein können, oder Naphthalin, Alkylfluoren oder Oxazol, wobei diese Gruppen gegebenenfalls ein- oder mehrfach mit L substituiert sind, wobei L F, Cl, Br oder eine Alkyl-, Alkoxy-, Alkylcarbonyl-, Alkylcarbonyloxy- oder Alkoxycarbonylgruppe mit 1 bis 12 C-Atomen bedeutet, worin ein oder mehrere H-Atome gegebenenfalls durch F oder Cl ersetzt sind, oder Thiophen, Thienothiophen oder Dithienothiophen, die durch ein oder mehrere Halogenatome substituiert sind.

8. Mono-, Oligo- oder Polymere nach mindestens einem der Ansprüche 1 bis 7, worin Ar¹ und/oder Ar² ausgewählt sind aus 1,4-Phenylen, fluoriertem 1,4-Phenylen, 2,5-Pyridin, 2,5-Pyrimidin, p,p'-Biphenyl, Naphthalin-2,6-diyl, Thiophen-2,5-diyl, fluoriertem oder alkyliertem Thiophen-2,5-diyl, fluoriertem Benzo-[1,2-b:4,5-b']dithiophen, 2,5-Thiazol, 2,5-Thiadiazol, 2,5-Oxazol und 2,5-Oxadiazol, die alle jeweils unsubstituiert oder ein- oder mehrfach mit L substituiert sind, wobei L F, Cl, Br oder eine Alkyl-, Alkoxy-, Alkylcarbonyl-, Alkylcarbonyloxy- oder Alkoxycarbonylgruppe mit 1 bis 12 C-Atomen bedeutet, worin ein oder mehrere H-Atome gegebenenfalls durch F oder Cl ersetzt sind.

9. Mono-, Oligo- oder Polymere nach Anspruch 8, worin Ar¹ und Ar² Thiophen-2,5-diyl bedeuten, das gegebenenfalls substituiert ist.

10. Mono-, Oligo- oder Polymere nach mindestens einem der Ansprüche 1 bis 9, worin Ar¹ und/oder Ar² eine Einfachbindung bedeuten.

11. Polymerisierbares Flüssigkristallmaterial enthaltend ein oder mehrere Mono-, Oligo- oder Polymere nach mindestens einem der vorhergehenden Ansprüche enthaltend mindestens eine polymerisierbare Gruppe, und gegebenenfalls enthaltend eine oder mehrere weitere polymerisierbare Verbindungen, worin mindestens eines/eine der Mono-, Oligo- und Polymere oder der weiteren polymerisierbaren Verbindungen mesogen oder flüssigkristallin ist.

12. Anisotrope Polymerfolie mit Ladungstransporteigenschaften, erhältlich aus einem polymerisierbaren Flüssigkristallmaterial nach Anspruch 11, welches in seiner Flüssigkristallphase in makroskopisch uniforme Ausrichtung orientiert und zur Fixierung des ausgerichteten Zustandes polymerisiert oder vernetzt ist.

13. Seitenketten-Flüssigkristallpolymer erhalten durch Polymerisation eines oder mehrerer Mono- oder Oligomere oder eines polymerisierbaren Materials nach mindestens einem der vorhergehenden Ansprüche oder durch Pfropfen eines oder mehrerer Mono- oder Oligomere oder eines polymerisierbaren Materials nach mindestens einem der vorhergehenden Ansprüche in einer polymeranalogen Reaktion auf ein Polymerrückgrat, gegebenenfalls mit einem oder mehreren zusätzlichen mesogenen oder nicht mesogenen Comonomeren.

14. Verwendung der Mono-, Oligo- und Polymere, polymerisierbaren Materialien und Polymere nach mindestens einem der vorhergehenden Ansprüche als Halbleiter oder Ladungstransportmaterialien in optischen, elektrooptischen oder elektronischen Vorrichtungen, wie zum Beispiel Feldeffekttransistoren (FET) als Komponenten von integrierten Schaltungen, von Dünnfilmtransistoren (thin film transistors - TFT) für Flachbildschirmanwendungen oder von RFID-Tags (radio frequency identification tags), oder in Halbleiterkomponenten für Anzeigen oder Anwendungen mit organischen Leuchtdioden (organic light emitting diode - OLED), darunter sowohl die Ladungstransport- als auch die Elektrolumineszentschicht.

15. Verwendung der Mono-, Oligo- oder Polymere, polymerisierbaren Materialien und Polymere nach mindestens einem der vorhergehenden Ansprüche als Elektrolumineszenzmaterialien, für OLED-Anwendungen wie z.B. Elektrolumineszenzanzeigen oder Hintergrundbeleuchtungen von Anzeigen, in Photovoltaik- oder Sensorvorrichtungen, als Elektrodenmaterialien in Batterien, als Photoleiter, für elektrophotographische Anwendungen wie elektrophotographische Aufzeichnung, für organische Speichervorrichtungen, zum Nachweis und zur Unterscheidung von DNA-Sequenzen und als Orientierungsschicht in LCD- oder OLED-Vorrichtungen.

16. Optische, elektrooptische oder elektronische Vorrichtung, FET, integrierte Schaltung (integrated circuit - IC), TFT, OLED oder Orientierungsschicht enthaltend ein Material, eine Komponente oder eine Vorrichtung nach mindestens einem der vorhergehenden Ansprüche.

17. TFT oder TFT-Anordnung für Flachbildschirme, RFID-Tag, Elektrolumineszenzanzeige oder Hintergrundbeleuchtung enthaltend ein Material, eine Komponente oder eine Vorrichtung nach mindestens einem der vorhergehenden Ansprüche oder einen FET, eine IC, einen TFT oder eine OLED nach Anspruch 16.

18. Sicherheitsmarkierung oder -vorrichtung enthaltend ein FET oder ein RFID-Tag nach Anspruch 17.

19. Mono-, Oligo- oder Polymer, Material oder Polymer nach mindestens einem der vorhergehenden Ansprüche, welches oxidativ oder reduktiv dotiert ist, so dass es leitende Ionenspezies bildet.

20. Ladungsinjektionsschicht, Planarisierungsschicht, Antistatikfolie oder leitendes Substrat oder leitende Struktur für Elektronikanwendungen oder Flachbildschirme, enthaltend ein Mono-, Oligo- oder Polymer, Material oder Polymer nach Anspruch 19.

## Revendications

1. Oligo- ou polymères comprenant une ou plusieurs unités récurrentes identiques ou différentes de la formule I 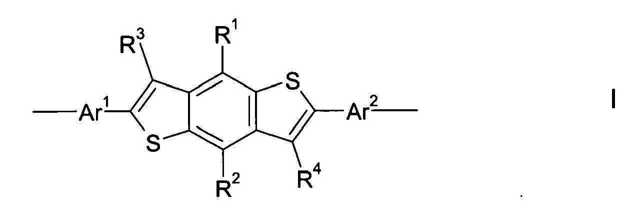 dans lesquels
R¹ à R⁴ sont indépendamment l'un de l'autre H, halogène ou alkyle en chaîne droite, ramifié ou cyclique avec 1 à 20 atomes de C, qui peuvent être non substitués, mono- ou poly-substitués par F, CI, Br, I ou CN, étant également possible que un ou plusieurs groupes CH₂ non adjacents soient remplacés, dans chaque cas indépendamment les uns des autres, par -O-, -S-, -NH-, -NR⁰-, -SiR⁰R⁰⁰-, -CO-, -COO-, -OCO-, -OCO-O-, -S-CO-, -CO-S-, -CH=CH- ou -C≡C- de telle sorte que des atomes de O et/ou S ne soient pas directement liés l'un à l'autre, ou aryle ou hétéroaryle optionnellement substitué, ou P-Sp-,
P est un groupe polymérisable ou réactif,
Sp est un groupe espaceur ou une liaison simple,
R⁰ et R⁰⁰ sont indépendamment l'un de l'autre H ou alkyle avec 1 à 12 atomes de C,
Ar¹ et Ar² sont indépendamment l'un de l'autre -CX¹=CX²-, -C≡C-, un groupe arylène ou hétéroarylène qui est optionnellement substitué avec un ou plusieurs R¹, ou une liaison simple,
X¹ et X² sont indépendamment l'un de l'autre H, F, Cl ou CN,
sous réserve que
a) dans au moins une unité récurrente, R¹ et R² sont différents de H et/ou R³ et R⁴ sont différents de H,
b) R¹ et R² ne représentent pas en même temps un groupe alcoxy,
et dans lesquels le nombre d'unités récurrentes est de 10 à 5000

2. Oligo- ou polymères selon la revendication 1, choisis parmi la formule I1 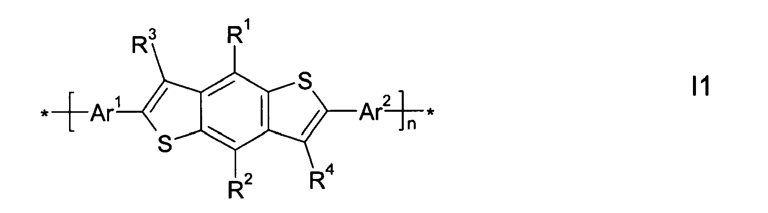 dans lesquels R¹, R², R³, R⁴, Ar¹ et Ar² ont indépendamment les uns des autres une des significations de la revendication 1, n est un entier de 10 à 5000, et dans lesquels les unités récurrentes peuvent être identiques ou différentes.

3. Oligo- ou polymères selon la revendication 2, choisis parmi la formule I1a 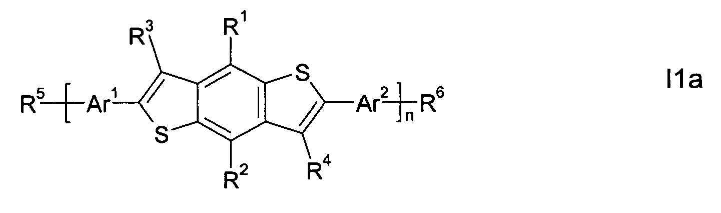 dans lesquels R¹, R², R³, R⁴, Ar¹, Ar² et n ont indépendamment les uns des autres une des significations de la revendication 2,
R⁵ et R⁶ sont indépendamment l'un de l'autre H, halogène, -Sn(R⁰)₃, -B(OR')(OR"), -CH₂Cl, -CHO, -CH=CH₂, -SiR⁰R⁰⁰R⁰⁰⁰, aryle ou hétéroaryle optionnellement substitué ou P-Sp-,
R⁰⁰⁰ a une des significations of R⁰ dans la revendication 1,
R' et R" sont indépendamment l'un de l'autre H ou alkyle avec 1 à 12 atomes de C, ou OR' et OR" ensemble avec l'atome de bore forment un groupe cyclique ayant 2 à 20 atomes de C, et
P, Sp, R⁰ et R⁰⁰ ont les significations de la revendication 1

4. Monomères de la formule I1a 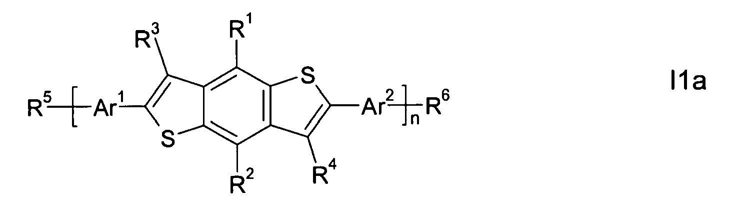 dans lesquels R¹, R², R³, R⁴, Ar¹ et Ar² ont indépendamment les uns des autres une des significations de la revendication 2, n est 1 et R⁵ et R⁶ sont choisis parmi Cl, Br, I, -Sn(R⁰)₃, -B(OR')(OR"), -CH₂Cl, -CHO et -SiR⁰R⁰⁰R⁰⁰⁰_{.}

5. Mono-, oligo- ou polymères selon au moins l'une des revendications 1 à 4, dans lesquels R¹ et R² sont choisis parmi C₁-C₂₀-alkyle qui est optionnellement substitué avec un ou plusieurs atomes de fluor, C₁-C₂₀-alkényle, C₁-C₂₀-alkynyle, C₁-C₂₀-thio-éther, C₁-C₂₀-silyle, C₁-C₂₀-ester, C₁-C₂₀-amino, C₁-C₂₀-fluoro-alkyle et aryle ou hétéroaryle optionnellement substitué, et R³ et R⁴ sont H.

6. Mono-, oligo- ou polymères selon au moins l'une des revendications 1 à 5, dans lesquels R³ et R⁴ sont choisis parmi C₁-C₂₀-alkyle qui est optionnellement substitué avec un ou plusieurs atomes de fluor, C₁-C₂₀-alkényle, C₁-C₂₀-alkynyle, C₁-C₂₀-thio-éther, C₁-C₂₀-silyle, C₁-C₂₀-ester, C₁-C₂₀-amino, C₁-C₂₀-fluoro-alkyle et aryle ou hétéroaryle optionnellement substitué, et R¹ et R² sont H.

7. Mono-, oligo- ou polymères selon au moins l'une des revendications 1 à 7, dans lesquels Ar¹ et/ou Ar² sont choisis parmi phényle dans lesquels, en addition, un ou plusieurs groupes CH peuvent être remplacés par N, ou naphtalène, alkyle fluorène ou oxazole, dans lesquels ces groupes sont optionnellement mono- ou polysubstitués avec L, dans lesquels L est F, CI, Br, ou un groupe alkyle, alcoxy, alkylcarbonyle, alkylcarbonyloxy ou alcoxycarbonyle avec 1 à 12 atomes de C, dans lesquels un ou plusieurs atomes de H are optionnellement remplacés par F ou CI, ou thiophène, thiénothiophène ou dithiénothiophène qui sont substitués par un ou plusieurs atomes d'halogène.

8. Mono-, oligo- ou polymères selon au moins l'une des revendications 1 à 7, dans lesquels Ar¹ et/ou Ar² sont choisis parmi 1,4-phénylène, 1,4-phénylène fluoré, 2,5-pyridine, 2,5-pyrimidine, p,p'-biphényle, naphtalène-2,6-diyle, thiophène-2,5-diyle, thiophène-2,5-diyle fluoré ou alkylaté, benzo[1,2-b:4,5-b']dithiophène fluoré, 2,5-thiazole, 2,5-thiadiazole, 2,5-oxazole et 2,5-oxadiazole, tous étant non substitués, mono- ou polysubstitués avec L, dans lesquels L est F, Cl, Br, ou un groupe alkyle, alcoxy, alkylcarbonyle, alkylcarbonyloxy ou alcoxycarbonyle avec 1 à 12 atomes de C, dans lesquels un ou plusieurs atomes de H sont optionnellement remplacés par F ou CI.

9. Mono-, oligo- ou polymères selon la revendication 8, dans lesquels Ar¹ et Ar² sont thiophène-2,5-diyle qui est optionnellement substitué.

10. Mono-, oligo- ou polymères selon au moins l'une des revendications 1 à 9, dans lesquels Ar¹ et/ou Ar² sont une liaison simple.

11. Matériau de cristal liquide polymérisable comprenant un ou plusieurs mono-, oligo- ou polymères selon au moins l'une des revendications précédentes comprenant au moins un groupe polymérisable, et comprenant optionnellement un ou plusieurs composés polymérisables supplémentaires, dans lequel au moins un desdits mono-, oligo- ou polymères ou composés polymérisables supplémentaires est cristallin liquide ou mésogène.

12. Film polymère anisotrope avec des propriétés de transport de charges pouvant être obtenu à partir d'un matériau de cristal liquide polymérisable selon la revendication 11 qui est aligné dans sa phase cristal liquide selon une orientation uniforme macroscopiquement et polymérisé ou réticulé pour fixer l'état orienté.

13. Polymère de cristal liquide en chaîne latérale obtenu par polymérisation d'un ou plusieurs mono- ou oligomères ou d'un matériau polymérisable selon au moins l'une des revendications précédentes ou par greffage d'un ou plusieurs mono- ou oligomères ou d'un matériau polymérisable selon au moins l'une des revendications précédentes à une ossature de polymère dans une réaction analogue à un polymère, optionnellement avec un ou plusieurs comonomères mésogènes ou non mésogènes additionnels.

14. Utilisation des mono-, oligo- ou polymères, matériaux polymérisables et polymères selon au moins l'une des revendications précédentes en tant que semiconducteurs ou matériaux de transport de charge dans des dispositifs optiques, électrooptiques ou électroniques, que transistors à effet de champ (FET) par exemple en tant que composants de circuit intégré, que transistors à film mince (TFT) pour des applications d'affichage à écran plat, ou qu'étiquettes d'identification radiofréquence (RFID), ou dans des composants semiconducteurs pour des affichages ou des applications de diodes émettrices de lumière organiques (OLED) incluant la couche à la fois électroluminescente et de transport de charges.

15. Utilisation des mono-, oligo- ou polymères, matériaux polymérisables et polymères selon au moins l'une des revendications précédentes en tant que matériaux électroluminescents, pour des applications OLED telles que des affichages électroluminescents ou des éclairages arrière d'affichages, dans des dispositifs photovoltaïques ou de capteurs, en tant que matériaux d'électrode dans des batteries, en tant que photoconducteurs, pour des applications électrophotographiques telles que des enregistrements électrophotographiques, pour des dispositifs de mémoire organiques, pour la détection et la discrimination de séquences ADN, et en tant que couche d'alignement dans des dispositifs LCD ou OLED.

16. Dispositif optique, électrooptique ou électronique, FET, circuit intégré (IC), TFT, OLED ou couche d'alignement comprenant un matériau, composant ou dispositif selon au moins l'une des revendications précédentes.

17. TFT ou réseau TFT pour des affichages à écran plat, étiquette d'identification radiofréquence (RFID), affichage ou éclairage arrière électroluminescent, comprenant un matériau, composant ou dispositif selon au moins l'une des revendications précédentes ou un FET, IC, TFT ou OLED selon la revendication 16.

18. Marquage ou dispositif de sécurité comprenant un FET ou une étiquette RFID selon la revendication 17.

19. Mono-, oligo- ou polymère, matériau ou polymère selon au moins l'une des revendications précédentes, qui est dopé de façon oxydante ou réductrice pour former des espèces ioniques de conduction.

20. Couche d'injection de charges, couche de planarisation, film antistatique ou substrat ou motif conducteur pour des applications électroniques ou pour des affichages à écran plat, comprenant un mono-, oligo- ou polymère, un matériau ou un polymère selon la revendication 19.
